# EUROPEAN PATENT APPLICATION

(11) **EP 0 943 613 A1**
(43) Date of publication of application: **22.09.1999**
(21) Application number: 97912512.7
(22) Date of filing: 19.11.1997
(51) Int. Cl.: C07D 319/18, C07D 405/06, C07D 405/12, A61K 31/335

(54) **OXYGENIC HETEROCYCLIC COMPOUNDS**

(30) Priority: 19.11.1996 JP 30778196
(71) Applicant: KYOWA HAKKO KOGYO KABUSHIKI KAISHA, Chiyoda-ku, Tokyo 100 (JP)
(72) Inventor: NAKASATO, Yoshisuke, Shizuoka 411-0943 (JP); OHSHIMA, Etsuo, Shizuoka 411 (JP); KOIKE, Rie, Shizuoka 411 (JP); ICHIMURA, Michio, Shizuoka 411 (JP); MANABE, Haruhiko, Shizuoka 411 (JP); SATO, Soichiro, Shizuoka 411 (JP); ISHII, Hidee, Shizuoka 410-21 (JP); YANAGAWA, Koji, Shizuoka 411-0943 (JP)
(74) Representative: VOSSIUS & PARTNER
(86) International application number: JP9704211
(87) International publication number: WO9822455

(57) **Abstract**

Oxygen-containing heterocyclic compounds represented by following Formula (I): 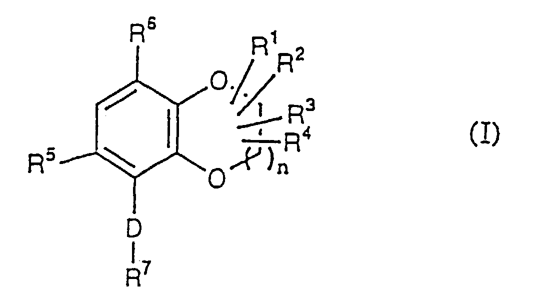
wherein n represents an integer of 1 to 4; R¹, R², R³ and R⁴ are the same or different and represent hydrogen, substituted or unsubstituted lower alkyl, or the like;
R⁵ represents hydrogen or halogen; R⁶ represents hydroxy or substituted or unsubstituted lower alkoxy; D represents (1) -C(R⁸)(R⁹)-X- or (2) a bond; R⁷ represents (1) substituted or unsubstituted lower alkyl, substituted or unsubstituted cycloalkyl, polycycloalkyl, substituted or unsubstituted aryl, substituted or unsubstituted aralkyl, a substituted or unsubstituted aromatic heterocyclic group, a substituted or unsubstituted heterocyclic group, or pyridine-*N*-oxide, (2) -Y-ZR¹⁴, (3) -Y-Z-(CH₂)ₘ-N(R^{16a})R^{16b}, or (4) -Y-CON(R^{17a})R^{17b}, or pharmaceutically acceptable salts thereof.

## Description

### Technical Field

The present invention relates to oxygen-containing heterocyclic compounds which exhibit phosphodiesterase (PDE) IV inhibitory activity and which are useful as therapeutic agents for inflammatory allergic diseases such as bronchial asthma, allergic rhinitis, and nephritis; autoimmune diseases such as rheumatoid arthritis, multiple sclerosis, Crohn's disease, psoriasis, and systemic lupus erythematosus; diseases of the central nervous system such as depression, amnesia, and dementia; organopathy associated with ischemic reflux caused by cardiac failure, shock, and cerebrovascular disease, and the like; insulin-resistant diabetes; wounds; AIDS, and the like.

### Background Art

Heretofore, it is known that the functions of numerous hormones and neurotransmitters are expressed by an increase in the concentration of adenosine 3',5'-cyclic monophosphate (cAMP) or guanosine 3',5'-cyclic monophosphate (cGMP), both of which are the secondary messengers in cells. The cellular concentrations of cAMP and cGMP are controlled by the generation and decomposition thereof, and their decomposition is carried out by PDE. Therefore, when PDE is inhibited, the concentrations of these secondary cellular messengers increase. Up to the present, 7 kinds of PDE isozymes have been found, and the isozyme-selective PDE inhibitors are expected to exhibit pharmacological effect based on their physiological significance and distribution *in vivo* [*TiPS*, 11, 150 (1990), *TiPS*, 12, 19 (1991)].

It is known that the activation of inflammatory leukocytes can be suppressed by increasing the concentration of the cellular cAMP. The activation of leukocytes causes secretion of inflammatory cytokines such as tumor necrosis factor (TNF), and expression of the cellular adhesion molecules such as intercellular adhesion molecules (ICAM), followed by cellular infiltration [*J. Mol. Cell. Cardiol*., 12 (Suppl. II), S61 (1989)].

It is known that the contraction of a respiratory smooth muscle can be suppressed by increasing the concentration of the cellular cAMP (*T. J. Torphy in Directions for New Anti-Asthma Drugs*, eds S. R. O'Donell and C. G. A. Persson, 1988, 37, Birkhauser-Verlag). The contraction of a respiratory smooth muscle is a main symptom of bronchial asthma. Infiltration of inflammatory-leukocytes such as neutrophils is observed in lesions of organopathy associated with ischemic reflux such as myocardial ischemia. It has been found that the type IV PDE (PDE IV) mainly participates in the decomposition of cAMP in these inflammatory cells and tracheal smooth muscle cells. Therefore, the inhibitors selective for PDE IV are expected to have therapeutic and/or preventive effect on inflammatory diseases, respiratory obstructive diseases, and ischemic diseases.

Further, the PDE IV inhibitors are expected to prevent the progress and spread of the inflammatory reaction transmitted by inflammatory cytokines such as TNFα and interleukin (IL)-8, because the PDE IV inhibitors suppress the secretion of these cytokines by increasing the concentration of cAMP. For example, TNFα is reported to be a factor of insulin-resistant diabetes because it declines the phosphorylating mechanism of insulin receptors of muscle and fat cells [*J. Clin. Invest*., 94, 1543-1549 (1994)]. Similarly, it is suggested that the PDE IV inhibitors are useful for these diseases because TNFα participates in the onset and progress of autoimmune diseases such as rheumatoid arthritis, multiple sclerosis, and Crohn's disease [*Nature Medicine*, 1, 211-214 (1995) and *ibid*., 244-248].

Drugs which increase cAMP are reported to enhance the healing of wounds [Nippon Yakuri-gakkai (Japanese Pharmacological Society), the 68th annual meeting (Nagoya), P3-116, 1995].

PDE IV-selective inhibitors having catechol structures are disclosed in WO96-00218, O96-00215, WO95-35285, WO95-35284, WO95-35283, WO95-35281, WO95-28926, WO95-27692, WO95-24381, WO95-22520, WO95-20578, WO95-17399, WO95-17392, WO95-14681, WO95-14680, WO95-14667, WO95-09837, WO95-09836, WO95-09627, WO95-09624, WO95-09623, WO95-08534, WO95-04046, WO95-04045, WO95-03794, WO95-01338, WO95-00516, WO95-00139, US5461056, EP0685479, P0685475, EP0685474, EP0671389, WO93-25517, WO94-25437, EP0623607, WO94-20446, WO94-20455 WO94-14800, WO94-14742, WO94-12461, WO94-10118, WO94-02465, WO93-19751, WO93-19750, WO93-19749, WO93-19748, WO93-19747, WO93-18024, WO93-15048, WO93-07141, Japanese Published Unexamined Patent Application No. 117239/93, WO92-19594, and EP0497564.

Japanese Published Unexamined Patent Application No. 242543/95 and Japanese Published Unexamined Patent Application No. 242655/95 disclose 2,3-dihydro-1,4-benzodioxine derivatives as therapeutic agents for hepatopathy.

WO92-10494 discloses 2,3-dihydro-1,4-benzodioxine derivatives having antagonist activity against serotonin (5HT)₃ receptors.

US5166367 discloses 2,3-dihydro-1,4-benzodioxine derivatives having anti-hallucination activity.

Japanese Published Unexamined Patent Application No. 179868/80 discloses 2,3-dihydro-1,4-benzodioxine derivatives having vasodilator activity.

AU521225 discloses 2,3-dihydro-1,4-benzodioxine derivatives as synthetic intermediates of cinnamoylpiperazine.

### Disclosure of the Invention

The present invention relates to oxygen-containing heterocyclic compounds represented by following Formula (I): 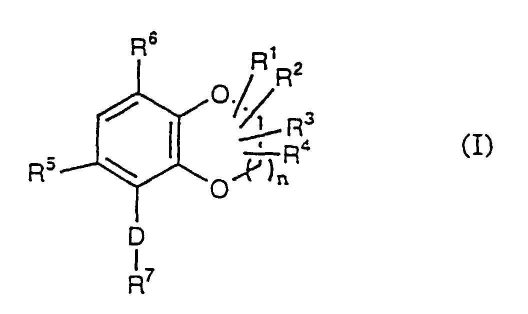 wherein n represents an integer of 1 to 4; R¹, R², R³ and R⁴ are the same or different and represent hydrogen, substituted or unsubstituted lower alkyl, substituted or unsubstituted cycloalkyl, polycycloalkyl, substituted or unsubstituted lower alkenyl, substituted or unsubstituted cycloalkenyl, substituted or unsubstituted aryl, a substituted or unsubstituted aromatic heterocyclic group, or substituted or unsubstituted aralkyl, or two groups present on the same carbon atom among R¹, R², R³ and R⁴ are combined to represent a saturated carbon ring, two groups present on the adjacent carbon atoms among R¹, R², R³ and R⁴ are combined to represent a saturated carbon ring together with the two carbon atoms adjacent thereto, or two groups present on the adjacent carbon atoms among R¹, R², R³ and R⁴ are combined to represent a single bond; R⁵ represents hydrogen or halogen; R⁶ represents hydroxy or substituted or unsubstituted lower alkoxy; D represents (1) -C(R⁸)(R⁹)-X- [wherein R⁸ and R⁹ are the same or different and represent hydrogen, substituted or unsubstituted lower alkyl, cycloalkyl, polycycloalkyl, substituted or unsubstituted lower alkenyl, cycloalkenyl, substituted or unsubstituted aryl, a substituted or unsubstituted aromatic heterocyclic group, hydroxy, substituted or unsubstituted lower alkoxy, lower alkanoyloxy, substituted or unsubstituted lower alkanoyl, cycloalkylcarbonyl, lower alkoxycarbonyl, cyano, or halogen, or R⁸ and R⁹ are combined to represent O, S or NR¹⁰ (wherein R¹⁰ represents hydrogen, substituted or unsubstituted lower alkyl, cycloalkyl, polycycloalkyl, substituted or unsubstituted aryl, a substituted or unsubstituted aromatic heterocyclic group, hydroxy, substituted or unsubstituted lower alkoxy, or lower alkanoyloxy); X represents -C(R¹¹)(R¹²)- (wherein R¹¹ and R¹² are the same or different and represent hydrogen, substituted or unsubstituted lower alkyl, cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano, or represent a single bond together with R⁸), S, NR¹³ (wherein R¹³ represents hydrogen, substituted or unsubstituted lower alkyl, cycloalkyl, substituted or unsubstituted aryl, a substituted or unsubstituted aromatic heterocyclic group, or substituted or unsubstituted aralkyl, or represents a single bond together with R⁸), or a bond], or (2) a bond; R⁷ represents (1) substituted or unsubstituted lower alkyl, substituted or unsubstituted cycloalkyl, polycycloalkyl, substituted or unsubstituted aryl, substituted or unsubstituted aralkyl, a substituted or unsubstituted aromatic heterocyclic group, a substituted or unsubstituted heterocyclic group, or pyridine-*N*-oxide, (2) -Y-ZR¹⁴ [wherein Y represents substituted or unsubstituted aryl, or a substituted or unsubstituted aromatic heterocyclic group; Z represents O, S, or NR¹⁵ (wherein R¹⁵ represents hydrogen, or substituted or unsubstituted lower alkyl, or represents a substituted or unsubstituted heterocyclic group together with R¹⁴); and R¹⁴ represents substituted or unsubstituted lower alkyl, substituted or unsubstituted aryl, or a substituted or unsubstituted aromatic heterocyclic group, or represents a substituted or unsubstituted heterocyclic group together with R¹⁵)], (3) -Y-Z-(CH₂)ₘ-N(R^{16a})R^{16b} (wherein Y and Z have the same meanings as defined above; R^{16a} and R^{16b} are the same or different and represent hydrogen, or substituted or unsubstituted lower alkyl, or R^{16a} and R^{16b} are combined to represent a substituted or unsubstituted heterocyclic group; and m represents an integer of 1 to 4), or (4) -Y-CON(R^{17a})R^{17b} (wherein Y has the same meaning as defined above; and R^{17a} and R^{17b} are the same or different and represent hydrogen, or substituted or unsubstituted lower alkyl, or R^{17a} and R^{17b} are combined to represent a substituted or unsubstituted heterocyclic group), or pharmaceutically acceptable salts thereof.

Hereinafter, the compounds represented by Formula (I) are referred to as Compounds (I). The same applies to the compounds of other formula numbers.

In the definitions of the groups in Formula (I), the lower alkyl and the lower alkyl moiety of the lower alkoxy, the lower alkanoyloxy, the lower alkanoyl, and the lower alkoxycarbonyl include straight-chain or branched alkyl groups having 1 to 8 carbon atoms, such as methyl, ethyl, propyl, isopropyl, butyl, isobutyl, sec-butyl, *tert*-butyl, pentyl, hexyl, heptyl, and octyl; the cycloalkyl and the cycloalkyl moiety of the cycloalkylcarbonyl include cycloalkyl groups having 3 to 10 carbon atoms, such as cyclopropyl, cyclobutyl, cyclopentyl, cyclohexyl, cycloheptyl, cyclooctyl, cyclononyl, and cyclodecyl; and the polycycloalkyl includes polycycloalkyl groups having 4 to 12 carbon atoms, such as bicyclo[3.2.1]octyl, bicyclo[4.3.2]undecyl, adamantyl, and noradamantyl. The lower alkenyl includes straight-chain or branched alkenyl groups having 2 to 8 carbon atoms, such as vinyl, 1-propenyl, allyl, methacryl, 1-butenyl, crotyl, pentenyl, isoprenyl, hexenyl, heptenyl, and octenyl; and the cycloalkenyl includes cycloalkenyl groups having 4 to 10 carbon atoms, such as cyclobutenyl, cyclopentenyl, cyclohexenyl, cycloheptenyl, cyclooctenyl, cyclononenyl, and cyclodecenyl. The aryl includes phenyl and naphthyl; and the aralkyl includes aralkyl groups having 7 to 15 carbon atoms, such as benzyl, phenethyl, benzhydryl, and naphthylmethyl.

The aromatic heterocyclic group includes 1 to 4 nitrogen atoms-containing 5- or 6-membered monocyclic aromatic heterocyclic groups, condensed bicyclic aromatic heterocyclic groups comprising a 5-membered ring and a 6-membered ring, and condensed bicyclic aromatic heterocyclic groups comprising a 6-membered ring and another 6-membered ring, such as pyridyl, pyrazinyl, pyrimidinyl, pyridazinyl, quinolinyl, isoquinolinyl, phthalazinyl, quinazolinyl, quinoxalinyl, naphthyridinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, and purinyl.

The heterocyclic group includes 5-, 6- or 7-membered monocyclic heterocyclic groups and condensed heterocyclic groups comprising a 6-membered ring and another 6-membered ring, such as pyrrolidinyl, piperidino, piperazinyl, morpholino, thiomorpholino, homopiperidino, homopiperazinyl, tetrahydropyridinyl, tetrahydroquinolinyl, and tetrahydroisoquinolinyl. The saturated carbon ring together with adjacent carbon atom and the saturated carbon ring together with two adjacent carbon atoms include saturated carbon rings having 3 to 10 carbon atoms, such as cyclopropane, cyclobutane, cyclopentane, cyclohexane, cycloheptane, cyclooctane, cyclononane, and cyclodecane. The halogen includes fluorine, chlorine, bromine, and iodine atoms.

The substituents in the substituted lower alkyl, substituted lower alkenyl, substituted cycloalkyl, substituted cycloalkenyl, and substituted lower alkanoyl are the same or different 1 to 3 substituents, such as lower alkyl, lower alkenyl, cycloalkyl, cycloalkenyl, hydroxy, lower alkoxy, carboxy, and halogen, in which the lower alkyl, lower alkenyl, cycloalkyl, cycloalkenyl, lower alkoxy, and halogen each have the same meanings as defined above.

The substituents in the substituted aryl, substituted aromatic heterocyclic group, substituted heterocyclic group, and substituted aralkyl are the same or different 1 to 3 substituents, such as lower alkyl hydroxy, lower alkoxy, lower alkanoyl, lower alkoxycarbonyl, carboxy, carbamoyl, trifluoromethyl, amino, mono- or di-lower alkyl substituted amino, cyano, nitro, and halogen. The lower alkyl, the lower alkyl moiety of the lower alkoxy, lower alkanoyl, lower alkoxycarbonyl, and mono- or di-lower alkyl substituted amino, and halogen each have the same meanings as defined above.

The substituents in the substituted lower alkoxy are the same or different 1 to 3 substituents, such as lower alkoxy and halogen, in which the lower alkoxy and halogen have the same meanings as defined above.

The pharmaceutically acceptable salts of Compound (I) include pharmaceutically acceptable acid addition salts, metal salts, ammonium salts, and organic amine addition salts.

The pharmaceutically acceptable acid addition salts of Compound (I) include inorganic acid addition salts such as hydrochloride, sulfate, nitrate, and phosphate, and organic acid addition salts such as acetate, maleate, fumarate, and citrate; the pharmaceutically acceptable metal salts include alkali metal salts such as a sodium salt and a potassium salt, alkaline earth metal salts such as a magnesium salt and a calcium salt, an aluminium salt, and a zinc salt; the pharmaceutically acceptable ammonium salts include ammonium and tetramethylammonium; and the pharmaceutically acceptable organic amine addition salts include an addition salt with morpholine or piperidine.

Processes for preparing Compound (I) are described below.

### Manufacturing method 1:

Compound (Ia), which is Compound (I) in which D is (1) -C(R⁸)(R⁹)-X-, can be obtained according to the following Processes 1-1 to 1-15.

### Process 1-1:

Compound (Iaa), which is Compound (Ia) in which X is -C(R¹¹)(R¹²)-, and R⁹ is hydrogen, substituted or unsubstituted lower alkyl, substituted or unsubstituted cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano, can be prepared according to the following reaction steps: 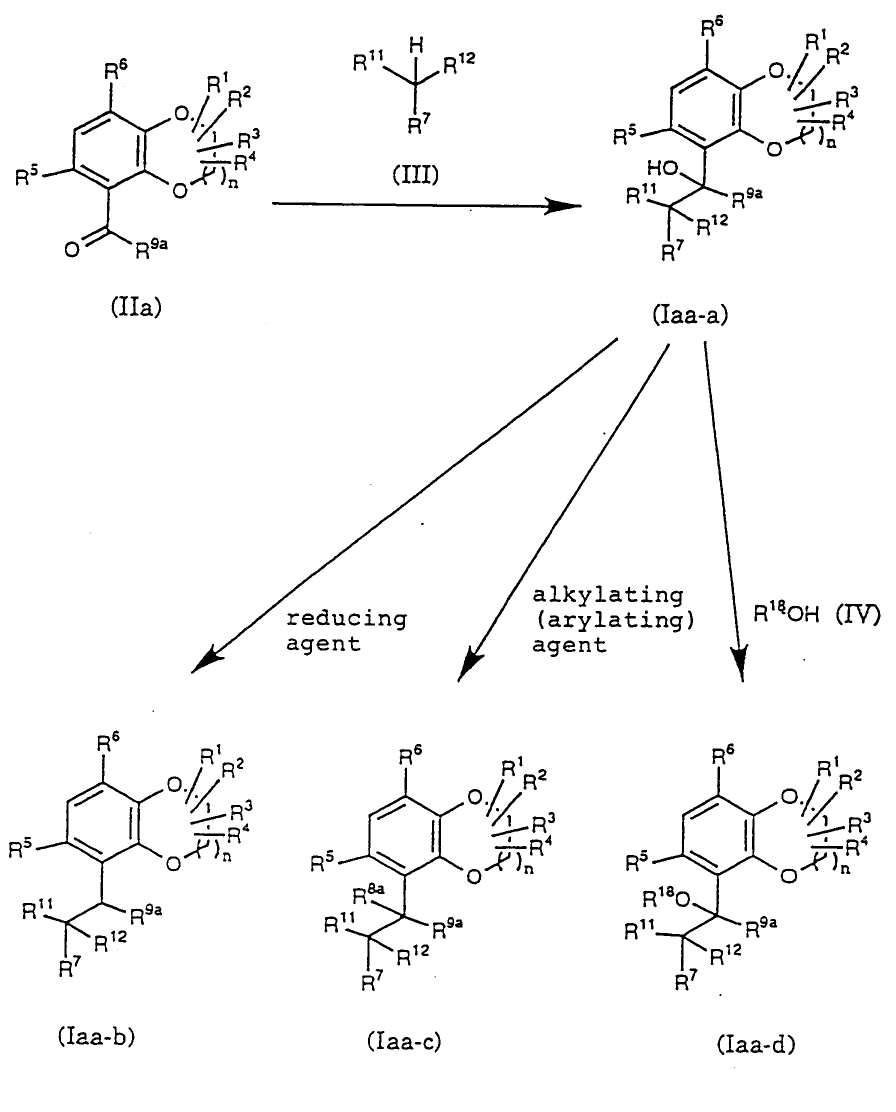 (In the formulae, R^{8a} is a group other than hydrogen, hydroxy, substituted or unsubstituted lower alkoxy, and lower alkanoyloxy in the definition of R⁸, and R^{8a} and R⁹ are not combined to form O, S, or NR¹⁰; R^{9a} is a group other than hydroxy, substituted or unsubstituted lower alkoxy, and lower alkanoyloxy in the definition of R⁹, and R^{9a} and R⁸ are not combined to form O, S, or NR¹⁰; R¹⁸ is substituted or unsubstituted lower alkyl, or substituted or unsubstituted lower alkanoyl; and R¹, R², R³, R⁴, R⁵, R⁶, R⁷, R¹¹, R¹², and n each have the same meanings as defined above.)

The substituted or unsubstituted lower alkyl and the substituted or unsubstituted lower alkanoyl in the definition of R¹⁸ each have the same meanings as defined above.

The starting Compound (IIa) can be obtained according to the methods in Reference Examples or similar methods thereto. In addition, the starting Compound (III) is commercially available, or, if the starting Compound (III) is a picoline derivative, it can be obtained according to a known method (WO94-20455) or a similar method thereto.

Compound (Iaa-a), which is Compound (Iaa) in which R⁸ is hydroxy, can be obtained by treating Compound (III) with a base in an inert solvent at the temperature between -100°C and room temperature for 5 minutes to 10 hours, followed by reaction with a starting Compound (IIa) at the temperature between -100°C and the boiling point of the employed solvent for 5 minutes to 30 hours.

Examples of the base are sodium hydroxide, potassium hydroxide, sodium methoxide, potassium ethoxide, sodium hydride, potassium hydride, butyl lithium, lithium diisopropylamide (LDA), potassium *tert*-butoxide, triethylamine, diisopropylethylamine, tributylamine, dicyclohexylmethylamine, *N*-methylmorpholine, *N*-methylpiperidine, diazabicycloundecene (DBU), and diazabicyclononene (DBN).

Examples of inert solvent are tetrahydrofuran (THF), dioxane, diethyl ether, ethylene glycol, triethylene glycol, glyme, diglyme, methanol, ethanol, butanol, isopropanol, dichloromethane, chloroform, benzene, toluene, dimethylformamide (DMF), and dimethyl sulfoxide (DMSO).

Compound (Iaa-b), which is Compound (Iaa) in which R⁸ is hydrogen, can be obtained by treating Compound (Iaa-a) with a reducing agent in the presence or absence of a catalytic amount to a largely excess amount of an acid catalyst in an inert solvent at the temperature between -100°C and the boiling point of the employed solvent for 5 minutes to 48 hours.

Examples of the acid catalyst are p-toluenesulfonic acid, methanesulfonic acid, hydrochloric acid, trifluoroacetic acid, boron trifluoride, aluminum chloride, stannic chloride, titanium tetrachloride, zinc chloride, and ferric chloride.

Examples of the reducing agent are triethylsilane, tributylsilane, dimethylphenylsilane, and trichlorosilane.

Examples of the inert solvent are THF, dioxane, diethyl ether, ethylene glycol, triethylene glycol, glyme, diglyme, dichloromethane, chloroform, benzene, and toluene.

Compound (Iaa-ba), which is Compound (Iaa-b) in which R¹² is hydrogen, can also be obtained by treating Compound (Iba) prepared by the method described below (Process 2-2) with a reducing agent in an inert solvent at the temperature between -100°C and the boiling point of the employed solvent for 5 minutes to 30 hours, or by subjecting Compound (Iba) to hydrogenation in the presence of a catalyst in an inert solvent at the temperature between room temperature and the boiling point of the employed solvent for 5 minutes to 30 hours. An example of the reducing agent is sodium borohydride; examples of the catalyst for the hydrogenation are palladium/carbon, palladium, platinum dioxide, and Raney nickel; and examples of the inert solvent are THF, dioxane, methanol, ethanol, butanol, and isopropanol.

Compound (Iaa-c), which is Compound (Iaa) in which R⁸ is a group other than hydrogen, hydroxy, substituted or unsubstituted lower alkoxy, and lower alkanoyloxy in the definition of R⁸, and R⁸ and R⁹ are not combined to form O, S, or NR¹⁰, can be obtained by reacting Compound (Iaa-a) with an alkylating (arylating) agent in the presence of an acid catalyst in an inert solvent at the temperature between -100°C and the boiling point of the employed solvent for 5 minutes to 30 hours.

Examples of the alkylating (arylating) agent are various kinds of alkyl- or arylmagnesium bromides, alkyl- or arylmagnesium chlorides, alkyl- or arylmagnesium iodides, trialkylaluminium, tetraalkyltitanium, dialkyltitanium chloride, Tebbe reagent, and trialkylsilylnitrile.

Examples of the acid catalyst are boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, chloroform, benzene, and toluene.

Compound (Iaa-d), which is Compound (Iaa) in which R⁸ is substituted or unsubstituted lower alkoxy or lower alkanoyloxy, can be obtained by reacting Compound (Iaa-a) with Compound (IV) in the presence of an acid catalyst, either in an inert solvent or in the absence of a solvent, at the temperature between -100°C and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, glyme, diglyme, dichloromethane, chloroform, benzene, toluene, DMF, and DMSO.

### Process 1-2:

Compound (Iab), which is Compound (Ia) in which X is S; R⁸ is hydrogen, substituted or unsubstituted lower alkyl, substituted or unsubstituted cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano; and R⁹ is hydrogen, substituted or unsubstituted lower alkyl, substituted or unsubstituted cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano, can be prepared by the following reaction steps: 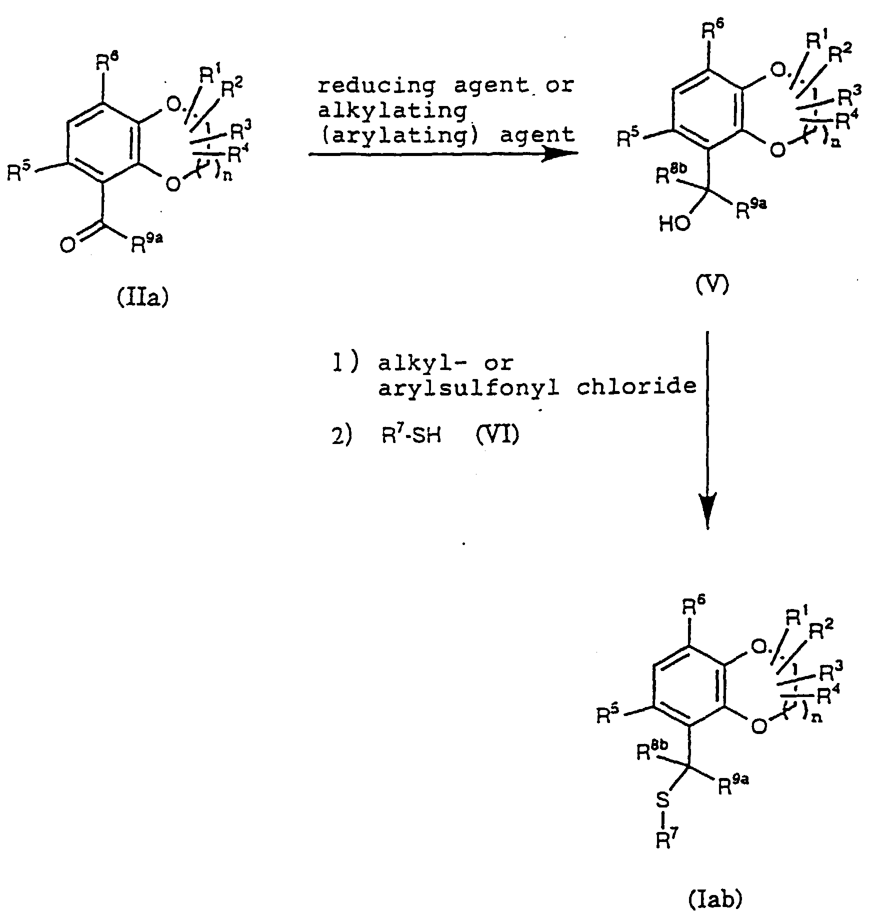 (In the formulae, R^{8b} is a group other than hydroxy, substituted or unsubstituted lower alkoxy, and lower alkanoyloxy in the definition of R⁸, and R^{8b} and R⁹ are not combined to form O, S, or NR¹⁰; and R¹, R², R³, R⁴, R⁵, R⁶, R⁷, R^{9a}, and n each have the same meanings as defined above.)

Compound (Va), which is Compound (V) in which R^{8b} is hydrogen, can be obtained by treating Compound (IIa) with a reducing agent in an inert solvent at the temperature between -100°C and the boiling point of the employed solvent for 5 minutes to 30 hours.

Examples of the reducing agent are lithium aluminium hydride and sodium borohydride.

Examples of the inert solvent are THF, dioxane, diethyl ether, ethylene glycol, triethylene glycol, glyme, diglyme, methanol, ethanol, butanol, isopropanol, dichloromethane, chloroform, benzene, and toluene.

Compound (Vb), which is Compound (V) in which R^{8b} is a group other than hydrogen in the definition of R^{8b}, can be obtained by reacting Compound (IIa) with an alkylating (arylating) agent in an inert solvent at the temperature between -100°C and the boiling point of the employed solvent for 5 minutes to 30 hours.

Examples of the alkylating (arylating) agent are various kinds of alkyl- or arylmagnesium bromides, alkyl- or arylmagnesium chlorides, alkyl- or arylmagnesium iodides, and various kinds of alkyl- or aryl lithiums.

Examples of the inert solvent are THF, dioxane, diethyl ether, glyme, diglyme, methanol, ethanol, butanol, isopropanol, dichloromethane, chloroform, benzene, and toluene.

Compound (Iab) can be obtained by reacting Compound (V) with, for example, an alkyl- or arylsulfonyl chloride, in the presence of a base in an inert solvent at the temperature between -20°C and 0°C for 5 minutes to 5 hours, followed by reaction with Compound (VI) at the temperature between 0°C and the boiling point of the employed solvent for 5 minutes to 48 hours.

Examples of the base are sodium hydride, potassium hydride, butyl lithium, LDA, potassium tert-butoxide, triethylamine, diisopropylethylamine, tributylamine, dicyclohexylmethylamine, *N*-methylmorpholine, *N*-methylpiperidine, DBU, and DBN.

Examples of the alkyl- or arylsulfonyl chloride are methanesulfonyl chloride, benzenesulfonyl chloride, and p-toluenesulfonyl chloride.

Examples of the inert solvent are THF, dioxane, diethyl ether, glyme, diglyme, dichloromethane, chloroform, benzene, toluene, DMF, and DMSO.

Alternatively, Compound (Iab) can also be obtained by reacting Compound (V) with Compound (VI) in the presence of an acid catalyst in an inert solvent at the temperature between -100°C and the boiling point of the employed solvent for 5 minutes to 48 hours.

Examples of the acid catalyst are p-toluenesulfonic acid, methanesulfonic acid, hydrochloric acid, trifluoroacetic acid, boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, chloroform, benzene, and toluene.

### Process 1-3:

Compound (Iac), which is Compound (Ia) in which X is NR¹³; R⁸ is hydrogen, substituted or unsubstituted lower alkyl, substituted or unsubstituted cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano; and R⁹ is hydrogen, substituted or unsubstituted lower alkyl, substituted or unsubstituted cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano, can be prepared by the following reaction step: 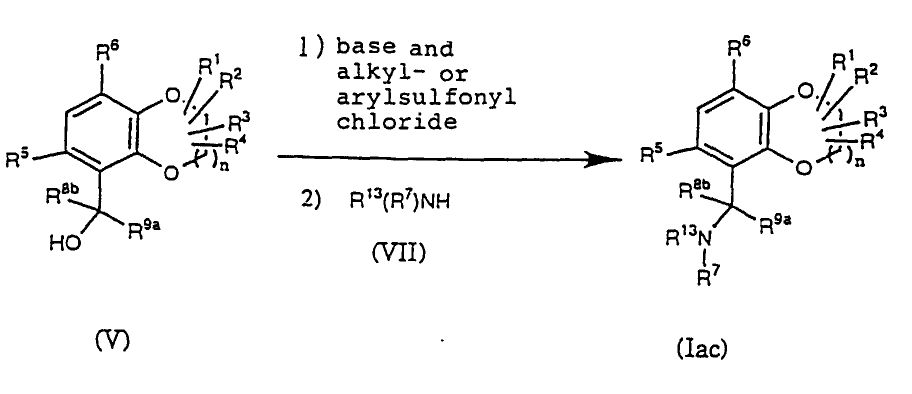 (In the formulae, R¹, R², R³, R⁴, R⁵, R⁶, R⁷, R^{8b}, R^{9a}, R¹³, and n each have the same meanings as defined above.)

Compound (Iac) can be obtained according to the method similar to the method described in Process 1-2 in which Compound (Iab) is obtained from Compound (V) and Compound (VI), using Compound (VII) instead of Compound (VI).

### Process 1-4:

Compound (Iad), which is Compound (Ia) in which D is -C(=O)-C(R¹¹)(R¹²)-, can be prepared by the following reaction step: 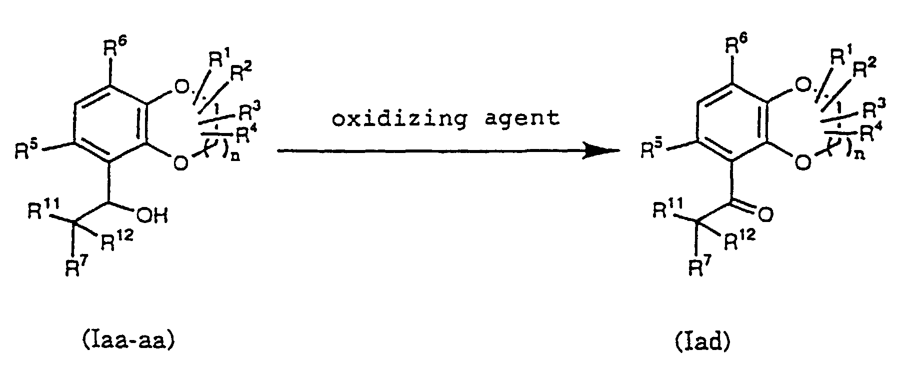 (In the formulae, R¹, R², R³, R⁴, R⁵, R⁶, R⁷, R¹¹, R¹², and n each have the same meanings as defined above.)

Compound (Iad) can be obtained treating Compound (Iaa-aa), which is Compound (Iaa-a) in which R^{9a} is hydrogen, with an oxidizing agent in an inert solvent containing water at the temperature between 0°C and the boiling point of the employed solvent for 5 minutes to 72 hours.

Examples of the oxidizing agent are manganese dioxide, potassium permanganate, pyridinium chlorochromate (PCC), and pyridinium dichromate (PDC).

Examples of the inert solvent are THF, dioxane, diethyl ether, ethylene glycol, triethylene glycol, glyme, diglyme, acetone, methyl vinyl ketone, dichloromethane, chloroform, benzene, toluene, DMF, and DMSO.

### Process 1-5:

Compound (Iad) can also be prepared according to the following reaction step: 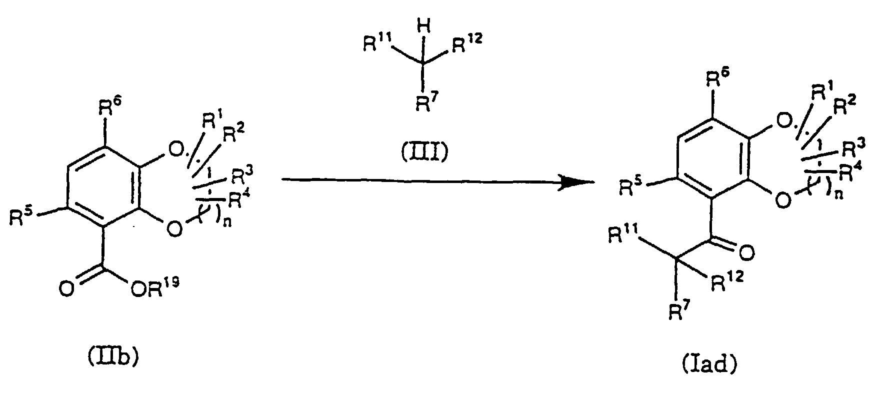 (In the formulae, R¹⁹ is substituted or unsubstituted lower alkyl; and R¹, R², R³, R⁴, R⁵, R⁶, R⁷, R¹¹, R¹², and n each have the same meanings as defined above.)

The substituted or unsubstituted lower alkyl in the definition of R¹⁹ has the same meaning as defined above.

The starting Compound (IIb) can be obtained according to the methods described in Reference Examples or similar methods thereto.

Compound (Iad) can be obtained according to the method similar to the method described in Process 1-1 in which Compound (Iaa-a) is obtained from Compound (IIa) and Compound (III), using Compound (IIb) instead of Compound (IIa).

### Process 1-6:

Compound (Iad) can also be prepared by the following reaction step: 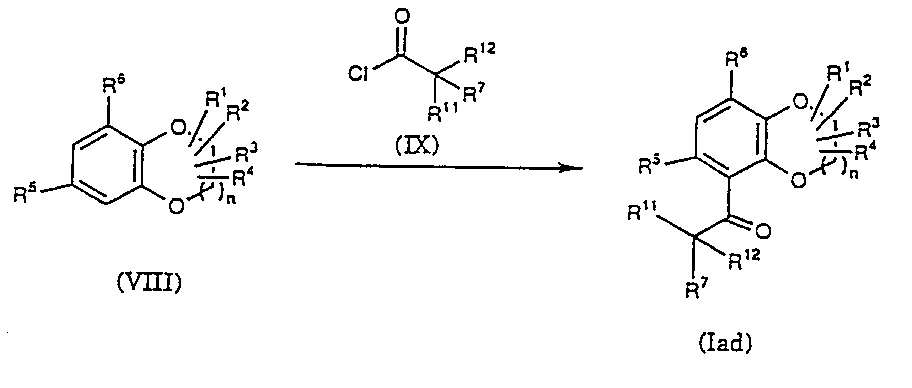 (In the formulae, R¹, R², R³, R⁴, R⁵, R⁶, R⁷, R¹¹, R¹², and n each have the same meanings as defined above.)

The starting Compound (VIII) can be obtained according to the methods described in Reference Examples or similar methods thereto.

Compound (Iad) can be obtained by reacting Compound (VIII) with Compound (IX) in the presence of an acid catalyst in an inert solvent at the temperature between -100°C and the boiling point of the employed solvent for 5 minutes to 30 hours.

Examples of the acid catalyst are boron trifluoride, aluminium chloride, stannic chloride, titanium tetrachloride, zinc chloride, and ferric chloride.

Examples of the inert solvent are THF, dioxane, diethyl ether, glyme, diglyme, dichloromethane, 1,2-dichloroethane, chloroform, benzene, nitrobenzene, and toluene.

### Process 1-7:

Compound (Iad) can also be prepared by the following reaction step: 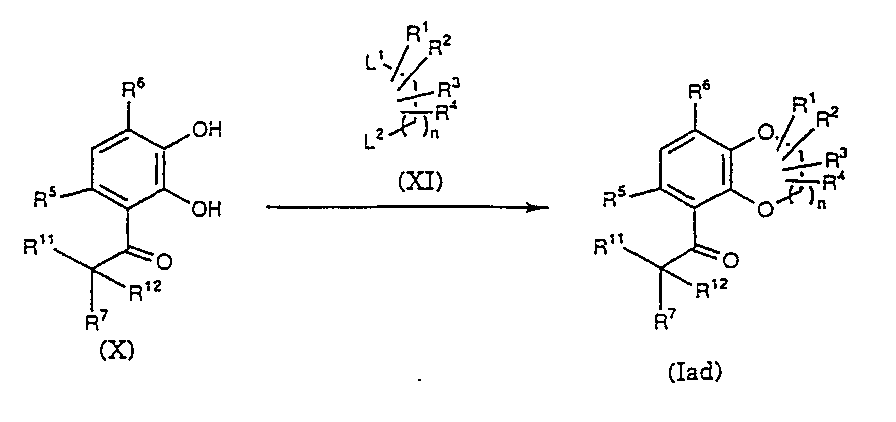 (In the formulae, L¹ and L² are the same or different and represent fluorine, chlorine, bromine or iodine; and R¹, R², R³, R⁴, R⁶, R⁷, R¹¹, R¹², and n each have the same meanings as defined above.)

The starting Compound (X) can be obtained according to the methods described in Reference Examples or similar methods thereto.

Compound (Iad) can be obtained by reacting Compound (X) with Compound (XI) in the presence of one equivalent to a largely excess amount of a base in an inert solvent at the temperature between 0°C and the boiling point of the employed solvent for 5 minutes to 48 hours.

Examples of the base are potassium carbonate, sodium carbonate, potassium fluoride, sodium hydroxide, potassium hydroxide, sodium methoxide, potassium ethoxide, sodium hydride, potassium hydride, butyl lithium, LDA, potassium tert-butoxide, triethylamine, diisopropylethylamine, tributylamine, dicyclohexylmethylamine, *N*-methylmorpholine, *N*-methylpiperidine, DBU, and DBN.

Examples of the inert solvent are pyridine, THF, dioxane, diethyl ether, dichloromethane, chloroform, benzene, toluene, xylene, acetone, dimethylacetamide, DMF, and DMSO.

### Process 1-8:

Compound (Iae), which is Compound (Ia) in which D is -C(=O)-NR¹³-, can be prepared by the following reaction step: 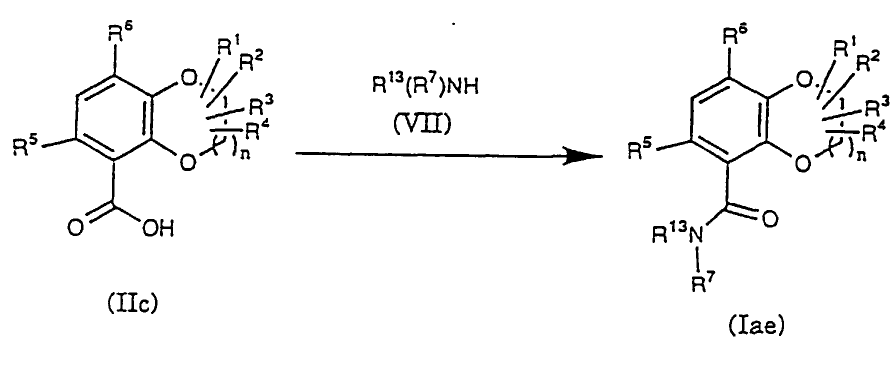 (In the formulae, R¹, R², R³, R⁴, R⁵, R⁶, R⁷, R¹³, and n each have the same meanings as defined above.)

The starting Compound (IIc) can be obtained according to the methods described in Reference Examples or similar methods thereto.

The desired Compound (Iae) can be obtained by dehydrative condensation between Compound (IIc) and Compound (VII). For the above condensation, numerous methods are known and applicable, as described in *Jikken* *Kagaku Koza*, 22, 137-172, the 4th edition [Nippon Kagakukai (Japanese Chemical Society), 1992]. For example, Compound (IIc) is treated with one equivalent to a largely excess amount of thionyl chloride, phosphorus pentachloride, oxalyl chloride, or the like, if necessary in the presence of a catalytic amount to 20 equivalents of a base, in an inert solvent at the temperature between 0°C and the boiling point of the employed solvent for 0.1 to 48 hours to give a corresponding acid chloride. Then, the desired Compound (Iae) can be obtained by reacting the obtained acid chloride with 0.5 to 50 equivalents of Compound (VII), if necessary in the presence of 0.5 equivalent to a largely excess amount of a base, in an inert solvent at the temperature between 0°C and the boiling point of the employed solvent for 0.1 to 48 hours.

Examples of the base are bases similar to those which are used in the manufacturing method for Compound (Iaa-a) described in Process 1-1.

Examples of the inert solvents are dichloromethane, chloroform, benzene, toluene, THF, dioxane, DMF, and DMSO.

### Process 1-9:

Compound (Iaf), which is Compound (Ia) in which D is -C(=O)-S-, can be prepared by the following reaction step: 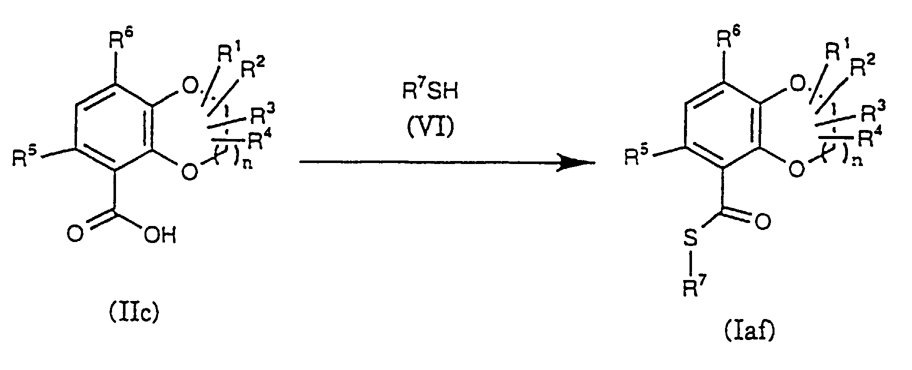 (In the formulae, R¹, R², R³, R⁴, R⁵, R⁶, R⁷, and n each have the same meanings as defined above.)

Compound (Iaf) can be obtained according to the method similar to the method described in Process 1-8 in which Compound (Iae) is obtained from Compound (IIc) and Compound (VII), using Compound (VI) instead of Compound (VII).

### Process 1-10:

Compound (Iag), which is Compound (Ia) in which D is -C(=S)-X-, can be prepared by the following reaction step: 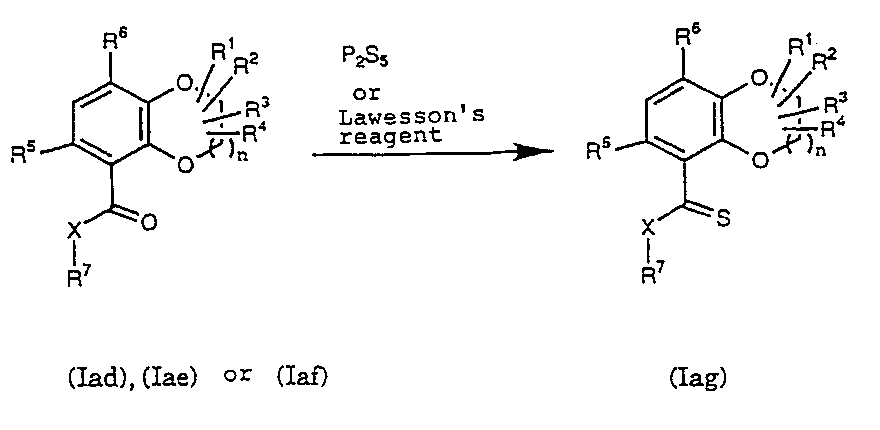 (In the formulae, R¹, R², R³, R⁴, R⁵, R⁶, R⁷, X, and n each have the same meanings as defined above.)

Compound (Iag) can be obtained by treating Compound (Iad), (Iae), or (Iaf) with phosphorus pentasulfide or Lawesson's reagent in an inert solvent at the temperature between room temperature and the boiling point of the employed solvent for 5 minutes to 72 hours.

Examples of the inert solvent are pyridine, THF, dioxane, diethyl ether, ethylene glycol, triethylene glycol, glyme, diglyme, dichloromethane, chloroform, benzene, toluene, xylene, DMF, and DMSO.

### Process 1-11:

Compound (Iah), which is Compound (Ia) in which D is -C(=NR¹⁰)-C(R¹¹)(R¹²)-, can be prepared by the following reaction step: 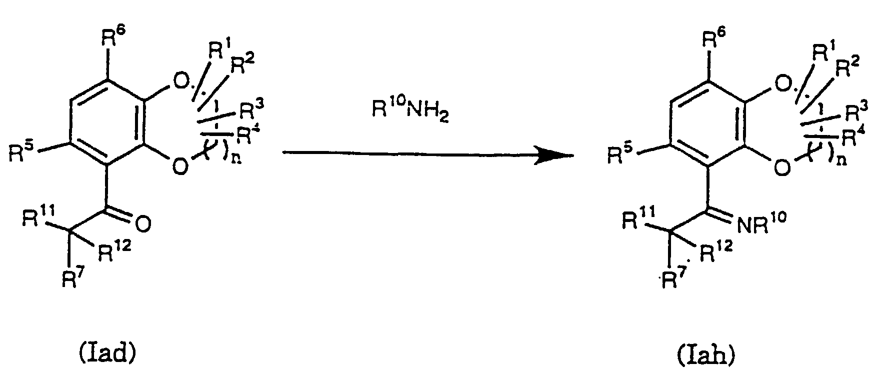 (In the formula, R¹, R², R³, R⁴, R⁵, R⁶, R⁷, R¹⁰, R¹¹, R¹² and n each have the same meanings as defined above.)

Compound (Iah) can be obtained by reacting Compound (Iad) with R¹⁰NH₂, if necessary, in the presence of an acid catalyst, either in an inert solvent or in the absence of a solvent at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, ethylene glycol, triethylene glycol, glyme, diglyme, methanol, ethanol, isopropanol, *tert*-butanol, dichloromethane, chloroform, benzene, toluene, DMF, DMSO, and pyridine.

### Process 1-12:

Compound (Iaj), which is Compound (Ia) in which D is D in Compound (Iaa), (Iad), or (Iae) in the definition of D, and R⁷ is pyridine-*N*-oxide, can be prepared by the following reaction step: 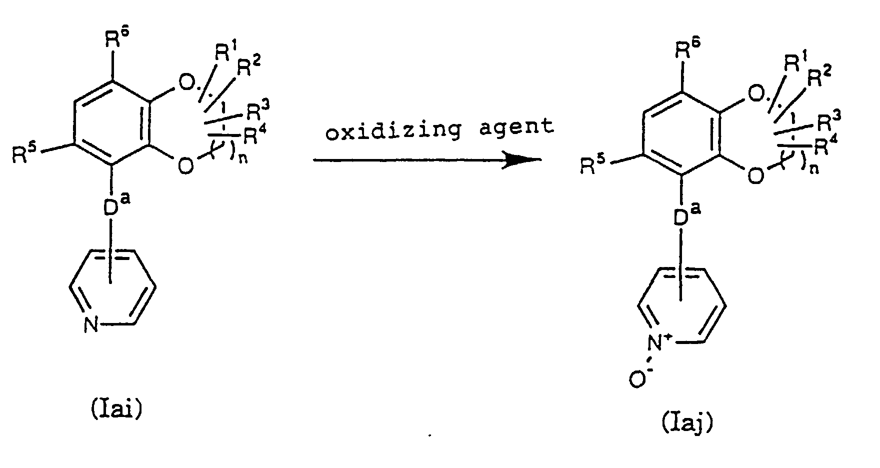 [In the formulae, D^{a} is D in Compound (Iaa), (Iad), or (Iae) in the definition of D; and R¹, R², R³, R⁴, R⁵, R⁶, and n each have the same meanings as defined above.]

Compound (Iaj) can be obtained by treating Compound (Iai), which is Compound (Ia) in which D is D in Compound (Iaa), (Iad), or (Iae) in the definition of D; and R⁷ is pyridyl, with an oxidizing agent in an inert solvent at the temperature between room temperature and the boiling point of the employed solvent for 5 minutes to 72 hours.

Examples of the inert solvent are dichloromethane, chloroform, benzene, toluene, xylene, DMF, DMSO, and acetic acid.

Amyl hydroperoxide and the like are exemplified.

### Process 1-13:

Compound (Iak), which is Compound (I) in which D is -C(R^{9a})=C(R¹²)-, can be prepared by the following reaction steps: 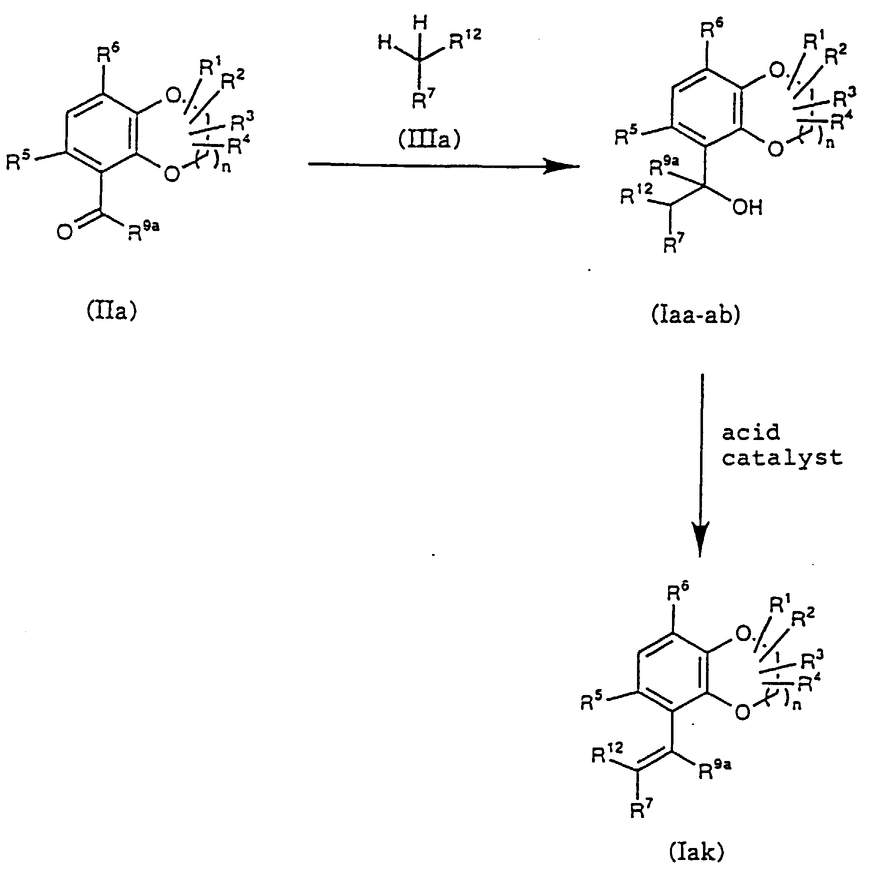 (In the formulae, R¹, R², R³, R⁴, R⁵, R⁶, R⁷, R^{9a}, R¹², and n each have the same meanings as defined above.)

Compound (Iaa-ab), which is Compound (Iaa-a) in which R¹¹ is hydrogen, can be obtained according to the method similar to the manufacturing method for Compound (Iaa-a) described in Process 1-1, using Compound (IIa) and Compound (IIIa), which is Compound (III) in which R¹¹ is hydrogen. Compound (Iaa-ab) is directly converted to Compound (Iak) without isolation when R¹² is substituted or unsubstituted lower alkanoyl, cycloalkylcarbonyl, lower alkoxycarbonyl, or cyano.

Compound (Iak) can be obtained by treating Compound (Iaa-ab) in the presence of an acid catalyst in an inert solvent at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl enter, ethylene glycol, triethylene glycol, glyme, diglyme, dichloromethane, chloroform, benzene, toluene, DMF, and DMSO.

### Process 1-14:

Compound (Iak) can also be prepared by the following reaction step: 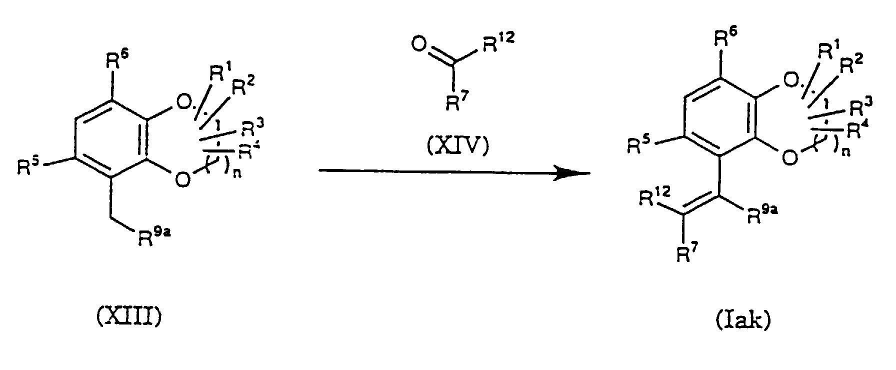 (In the formulae, R¹, R², R³, R⁴, R⁵, R⁶, R⁷, R^{9a}, R¹², and n each have the same meanings as defined above.)

The starting Compound (XIII) can be obtained according to the methods described in Reference Examples or similar methods thereto.

Compound (Iak) can be obtained by treating the starting Compound (XIII) with a base in an inert solvent at the temperature between -100°C and the boiling point of the employed solvent for 5 minutes to 10 hours, followed by reaction with Compound (XIV) at the temperature between -100°C and the boiling point of the employed solvent for 5 minutes to 30 hours.

Examples of the base and the invert solvent are bases and invert solvents similar to those which are used in the manufacturing method for compound (Iaa-a) described in Process 1-1.

### Process 1-15:

Compound (Ial), which is Compound (I) in which D is -C(R^{9a})=N-, can be prepared by the following reaction steps: 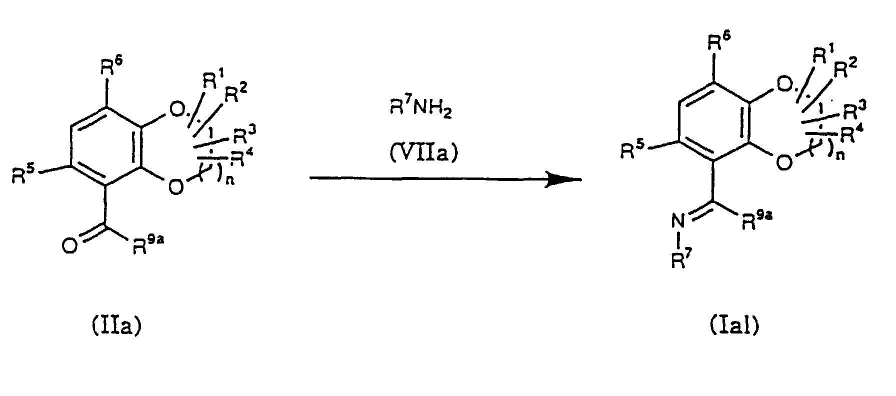 (In the formulae, R¹, R², R³, R⁴, R⁵, R⁶, R⁷, R^{9a}, and n each have the same meanings as defined above.)

Compound (Ial) can be obtained by reacting Compound (IIa) and Compound (VIIa), which is Compound (VII) in which R¹³ is hydrogen in the presence of an acid catalyst, either in an inert solvent or in the absence of a solvent, at the temperature between room temperature and the boiling point of the employed solvent for 5 minutes to 48 hours.

Examples of the acid catalyst are p-toluenesulfonic acid, methanesulfonic acid, hydrochloric acid, sulfuric acid, acetic acid, and trifluoroacetic acid.

Examples of the inert solvent are THF, dioxane, diethyl ether, ethylene glycol, triethylene glycol, glyme, diglyme, methanol, ethanol, isopropanol, tert-butanol, dichloromethane, chloroform, benzene, toluene, DMF, and DMSO.

### Manufacturing method 2:

Compound (Ib), which is Compound (I) in which D is a bound, can be obtained by the following processes 2-1 or 2-2: 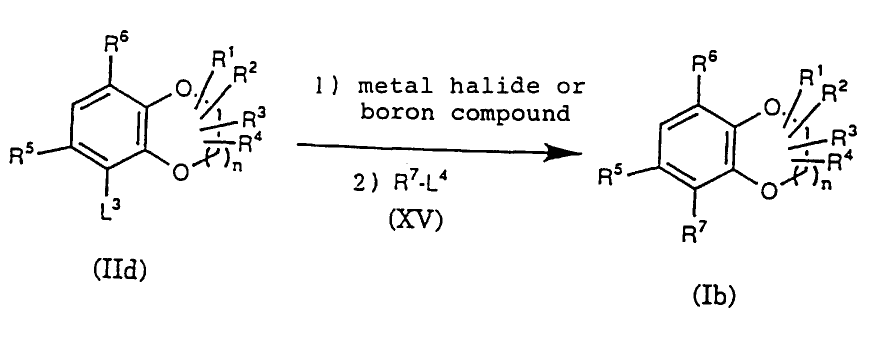 (In the formulae, L³ and L⁴ are the same or different and represent iodine, bromine, or chlorine; and R¹, R², R³, R⁴, R⁵, R⁶, R⁷, and n each have the same meanings as defined above.)

Compound (Ib) can be obtained by treating Compound (IId) with a base in an inert solvent at the temperature between -100°C and room temperature for 5 minutes to 10 hours, followed by reaction with a metal halide or a boron compound at the temperature between -100°C and the boiling point of the employed solvent for 5 minutes to 30 hours, and then reacting the obtained product with Compound (XV) in the presence of a catalytic amount to a largely excess amount of a palladium complex in an inert solvent at the temperature between room temperature and the boiling point of the employed solvent for 5 minutes to 30 hours. Moreover, if necessary, an oxidizing agent, such as salts (e.g., lithium chloride) or silver oxide, may be added in the case of the reaction with Compound (XV).

Examples of the metal halide are alkyltin halides such as tributyltin chloride and trimethyltin chloride, and zinc halides such as zinc chloride, zinc bromide, and zinc iodide; and examples of the boron compound are trimethoxy boron, phenylboric acid, and boric acid.

Examples of the palladium complex are tetrakis(triphenylphosphine)palladium, bis(triphenylphosphine)palladium dichloride, bis(acetonitrile)palladium dichloride, and palladium acetate.

Examples of the base are sodium hydroxide, potassium hydroxide, sodium methoxide, potassium ethoxide, sodium hydride, potassium hydride, butyl lithium, LDA, potassium tert-butoxide, triethylamine, diisopropylethylamine, tributylamine, dicyclohexylmethylamine, *N*-methylmorpholine, *N*-methylpiperidine, DBU, and DBN.

Examples of the inert solvent employed in the treatment with a base followed by reaction with a metal halide or a boron compound are THF, dioxane, diethyl ether, ethylene glycol, triethylene glycol, glyme, diglyme, methanol, ethanol, butanol, isopropanol, dichloromethane, chloroform, benzene, toluene, DMF, and DMSO.

Examples of the inert solvent employed in the reaction with Compound (XV) are THF, dioxane, diethyl ether, dichloromethane, chloroform, benzene, toluene, dimethylacetamide (DMA), DMF, and DMSO.

### Process 2-2:

Compound (Icb), which is Compound (Ib) in which R⁷ is R^{7b} which is aryl substituted with a carboxyl group or an aromatic heterocyclic group substituted with a carboxyl group, can be prepared by the following process: 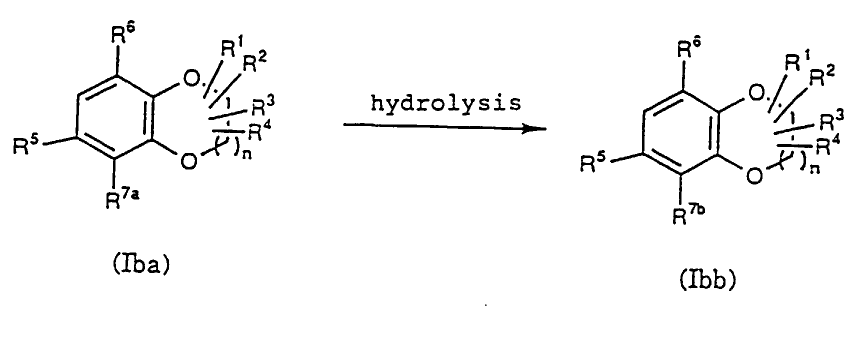 (In the formulae, R^{7a} is aryl substituted with lower alkoxycarbonyl or a cyano group, or an aromatic heterocyclic group substituted with lower alkoxycarbonyl or a cyano group; R^{7b} is aryl substituted with a carboxyl group, or an aromatic heterocyclic group substituted with a carboxyl group; and R¹, R², R³, R⁴, R⁵, R⁶ and n each have the same meanings as defined above.)

The lower alkoxycarbonyl, aryl and aromatic heterocyclic group in the definition of R^{7a} have the same meanings as those described above, respectively. The aryl and heterocyclic group in the definition of R^{7b} have the same meanings as those described above, respectively.

Compound (Ibb) can be obtained by treating Compound (Iba) in the presence of a catalytic amount to a largely excess amount of a base in an inert solvent containing water at the temperature between room temperature and the boiling point of the employed solvent for 0.1 to 48 hours.

Examples of the base are sodium hydroxide, potassium hydroxide, sodium methoxide, potassium ethoxide, sodium hydride, potassium hydride, butyl lithium, LDA, potassium *tert*-butoxide, triethylamine, diisopropylethylamine, tributylamine, dicyclohexylmethylamine, *N*-methylmorpholine, *N*-methylpiperidine, DBU, and DBN.

Examples of the inert solvent are THF, dioxane, diethyl ether, ethylene glycol, triethylene glycol, glyme, diglyme, methanol, ethanol, butanol, isopropanol, dichloromethane, chloroform, benzene, toluene, dimethylacetamide (DMA), DMF, and DMSO.

### Manufacturing method 3:

Compound (Ic), which is Compound (I) in which R⁷ is -Y-ZR¹⁴ or -Y-Z-(CH₂)ₘ-NR^{16a}R^{16b}, can be obtained by the following process: 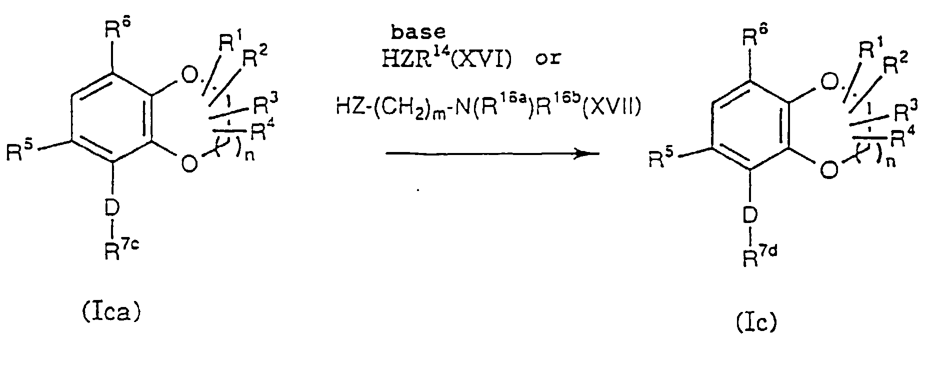 (In the formulae, R^{7c} is aryl substituted with 1 to 3 halogens or an aromatic heterocyclic group substituted with 1 to 3 halogens; R^{7d} is -Y-ZR¹⁴ or -Y-Z-(CH₂)ₘ-NR^{16a}R^{16b}; and R¹, R², R³, R⁴, R⁵, R⁶, R¹⁴, R^{16a}, R^{16b}, D, Y, Z, m, and n each have the same meanings as defined above.)

The aryl and aromatic heterocyclic group in the definition of R^{7c} have the same meanings as those described above, respectively.

Compound (Ic) can be obtained by reacting Compound (Ica) with Compound (XVI) or Compound (XVII) in the presence of a base in an inert solvent at the temperature between room temperature and the boiling point of the employed solvent for 0.1 to 48 hours.

Examples of the base are sodium hydroxide, potassium hydroxide, sodium methoxide, potassium ethoxide, sodium hydride, potassium hydride, butyl lithium, LDA, potassium *tert*-butoxide, triethylamine, diisopropylethylamine, tributylamine, dicyclohexylmethylamine, *N*-methylmorpholine, *N*-methylpiperidine, DBU, and DBN.

Examples of the inert solvent are THF, dioxane, diethyl ether, ethylene glycol, triethylene glycol, glyme, diglyme, methanol, ethanol, butanol, isopropanol, dichloromethane, chloroform, benzene, toluene, dimethylacetamide (DMA), DMF, and DMSO.

The intermediates and the desired compounds in the processes described above can be isolated and purified by purification methods conventionally used in synthetic organic chemistry, for example, filtration, extraction, washing, drying, concentration, recrystallization, and various kinds of chromatography. The intermediates may also be subjected to the subsequent reaction without purification.

Compound (I) can exist in the form of stereoisomers such as geometrical isomers and optical isomers, and the present invention covers all isomers including these isomers and mixtures thereof.

In the case where a salt of Compound (I) is desired and it is produced in the form of the desired salt, it can be subjected to purification as such. In the case where Compound (I) is produced in the free form and its salt is desired, Compound (I) is dissolved or suspended in a suitable solvent, followed by addition of an acid or a base to form a salt, which may be isolated and purified.

Compound (I) and pharmaceutically acceptable salts thereof may be in the form of adducts with water or various solvents, which are also within the scope of the present invention.

Examples of Compound (I) obtained in the present invention are shown in Tables 1 and 2.

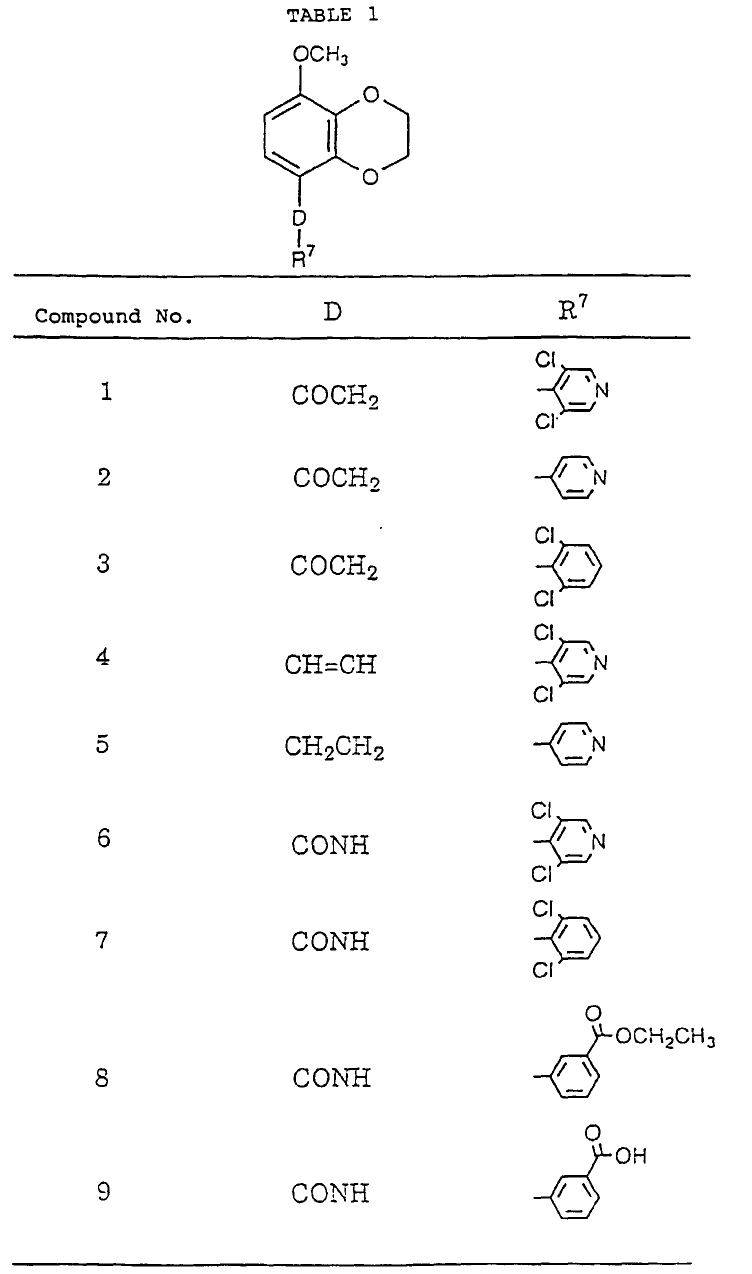

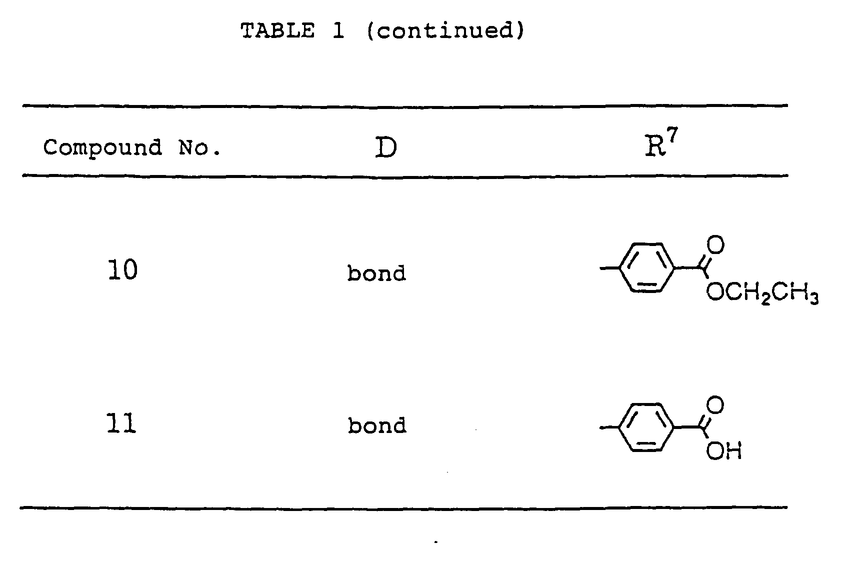

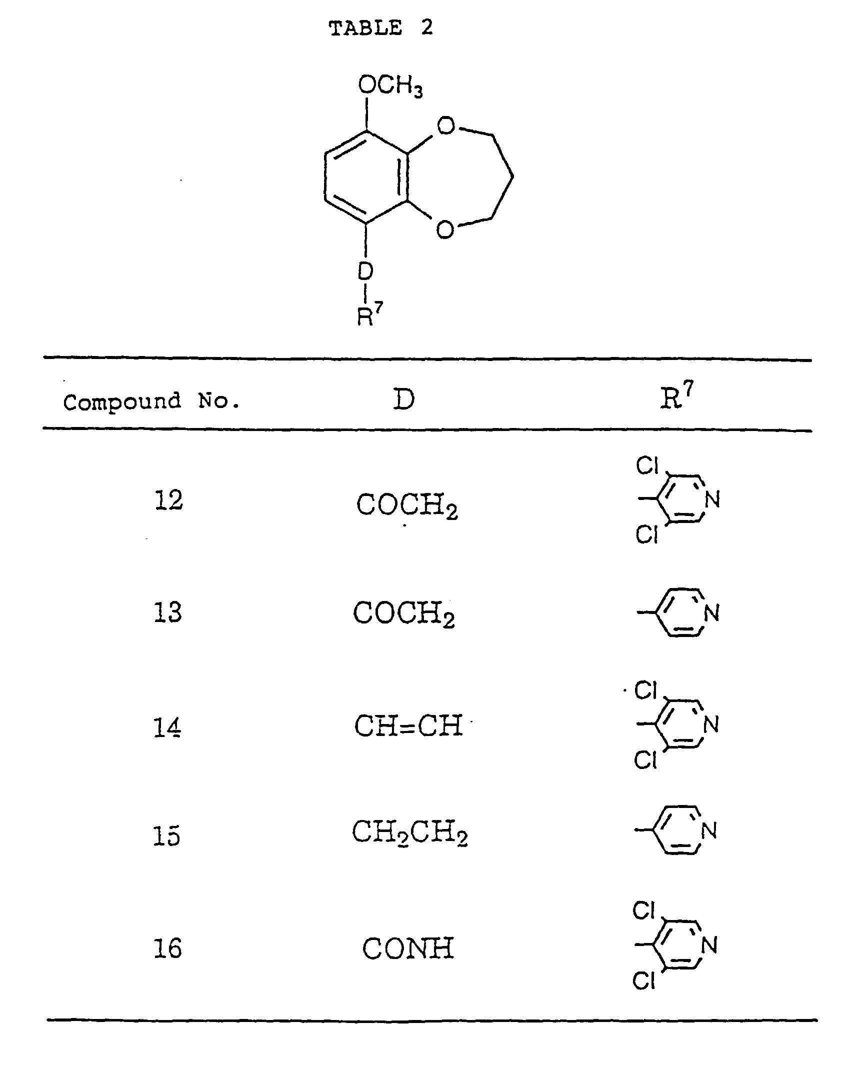

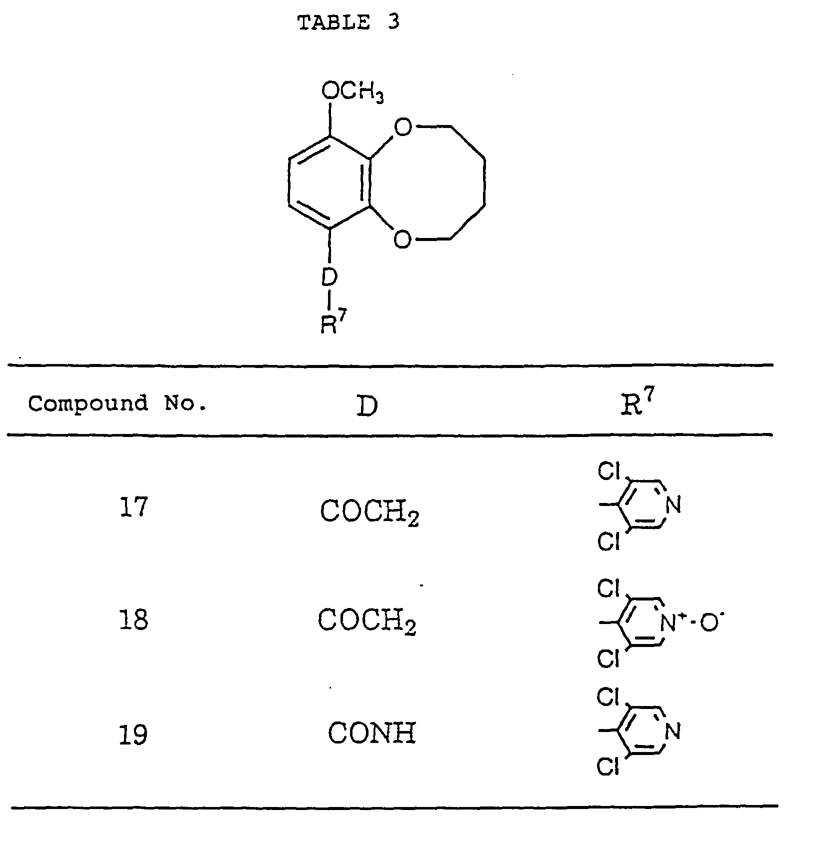

The pharmacological activities of the representative Compounds (I) are described in more detail by Test Examples.

### Test Example 1 Inhibition of the PDE IV Enzyme derived from a Dog Trachea

cAMP-specific phosphodiesterase (PDE IV) was isolated and purified from a dog tracheal smooth muscle according to the method of Torphy *et al*. [*Molecular Pharmacol*., 37, 206-214 (1990)]. The PDE activity was measured by the following two steps according to the method of Kincaid and Manganiello *et al*. [*Method in Enzymology* (J. D. Corbin and R. A. Jonson, Eds.) 199, 457-470 (1988)]. [³H]cAMP (final concentration: 1 µM) was used as a substrate, and the reaction was carried out in a standard mixture solution containing *N*,*N*-bis(2-hydroxyethyl)-2-aminoethanesulfonic acid (50 mM, pH=7.2), MgCl₂ (1 mM), and soybean trypsin inhibitor (0.1 mg/ml). The reaction was initiated by adding the enzyme, and the mixture was incubated at 30°C for 10 to 30 minutes. After stopping the reaction with hydrochloric acid, the generated 5'-AMP was completely decomposed by 5'-nucleotidase. Chromatography was carried out using DEAE-Sephadex A-25, and the eluted [³H]adenosine was counted using a scintillation counter. Each of the test drugs was dissolved in DMSO (concentration: 1.7%) and then added to the mixture.

In this study, Compounds 1 and 6 showed enzyme inhibitory activity of over 50% at 1 µm.

### Test Example 2 Recombinant human PDE IVA enzyme inhibitory test

The cDNA encoding human phosphodiesterase (HSPDE4A) was isolated from human testis. The predicted amino acid sequence is an amino acid sequence which lacks 223 amino acids potion at the N-terminus in the sequence (HSPD4A5) previously reported by Bolger, G. *et al*. [*Mol. Cell. Biol*., 13, 6558-6571 (1993)]. This recombinant protein was expressed by means of the expression plasmid *E. coli*, and purified. The PDE activity was measured by the following two-steps according to the method of Kincaid, R., L. and Manganiello, V., C. [*Method. Enzymol*., 159, 457-470 (1988)]. [³H]cAMP (final concentration: 1 mmol/L) was used as a substrate, and the reaction was carried out in a standard mixture solution containing *N*,*N*-bis(2-hydroxyethyl)-2-aminoethansulfonic acid (50 mmol/L, pH 7.2), MgCl₂ (1 mmol/L), and soybean trypsin inhibitor (0.1 mg/mL). The reaction was initiated by addition of the enzymes, and the mixture was incubated at 30°C for 10 to 30 minutes. After stopping the reaction with hydrochloric acid, the generated 5'-AMP was completely decomposed by 5'-nucleotidase. Chromatography was carried out using DEAE-Sephadex A-25, and the eluted [³H]adenosine was counted using a scintillation counter. Each of the test drugs was dissolved in DMSO (final concentration: 1.7%) and then added to the mixture.

In this study, the enzyme inhibitory activities of Compounds 12, 14, 17 and 18 in the Examples at 10⁻⁷ M are shown in Table 4.

**Table 4**

| Enzyme Inhibitory Activity against Human Phosphodiesterase IVA (hPDE-IVA) | |
|---|---|
| Compound No. | hPDE-IVA Enzyme Inhibitory Activity (%) (10⁻⁷M) |
| 12 | 84 |
| 14 | 84 |
| 17 | 92 |
| 18 | 93 |

Although Compound (I) or pharmaceutically acceptable salts thereof may be administered as they are, it is usually desirable to provide them in the form of various pharmaceutical preparations. Such pharmaceutical preparations may be used for animals and human beings.

The pharmaceutical preparations in accordance with the present invention may contain Compound (I) or a pharmaceutically acceptable salt thereof, as an active ingredient, either solely or as a mixture with other therapeutically effective components. The pharmaceutical preparations may be prepared by any means which are well known in the technical field of pharmaceutics after mixing the active ingredient with one or more pharmaceutically acceptable carriers.

It is desired to use the administration route which is the most effective in therapy such as an oral route or a parenteral route which includes intrabuccal, intratracheal, intrarectal, subcutaneous, intramuscular, and intravenous administrations.

Example of the dosage form are nebulae, capsules, tablets, granules, powders, syrups, emulsions, suppositories, injections, ointments, and tapes.

Liquid preparations suitable for oral administration such as emulsions and syrups can be prepared using water; sugars such as sucrose, sorbitol, and fructose; glycols such as polyethylene glycol and propylene glycol; oils such as sesame oil, olive oil, and soybean oil; preservatives such as p-hydroxybenzoates; flavors such as strawberry flavor and peppermint; and the like. Capsules, tablets, powders, granules, and the like can be prepared using excipients such as lactose, glucose, sucrose, and mannitol; disintegrators such as starch and sodium alginate; lubricants such as magnesium stearate and talc; binders such as polyvinyl alcohol, hydroxypropyl cellulose, and gelatin; surfactants such as fatty acid esters; plasticizers such as glycerin; and the like.

Preparations suitable for parenteral administration comprise sterilized aqueous preparations of the active compound which are preferably isotonic to the blood of the patient. For example, a solution for injection is prepared using a carrier such as a salt solution, a glucose solution, or a mixture of a salt solution and a glucose solution. Preparations for intrarectal administration are prepared using a carrier such as cacao fat, hydrogenated fat, or a hydrogenated carboxylic acid, and provided as suppositories. Nebulae are prepared using an active compound *per se* or with carriers which can disperse the active compound as fine particles to facilitate absorption without stimulating oral or respiratory mucosa. Practical examples of the carrier are lactose and glycerin. Preparations such as aerosols and dry powders can be used depending upon the properties of the active compound and the employed carriers.

These parenteral preparations may also contain one or more auxiliary components selected from glycols, oils, flavors, preservatives, excipients, disintegrators, lubricants, binders, surfactants, and plasticizers, all of which are mentioned in the above oral preparations.

The effective dose and the administration schedule of Compound (I) or pharmaceutically acceptable salts thereof may vary depending upon the administration route, age and body weight of the patient, and the type or degree of the disease to be treated, but usually, in the case of oral administration, the effective compound is administered in a dose of 0.01 mg to 1 g/adult/day, preferably 0.05 to 50 mg/adult/day, at one time or in several parts. In the case of parenteral administration such as intravenous injection, the effective compound is administered in a dose of 0.001 to 100 mg/adult/day, preferably 0.01 to 10 mg/adult/day, at one time or in several parts. These doses should, however, vary depending upon various conditions as given above.

Certain embodiments of the present invention are illustrated in the following examples and reference examples.

### Best Mode for Carrying Out the Invention

### Example 1

### 5-[2-(3,5-Dichloro-4-pyridyl)-1-oxoethyl]-2,3-dihydro-8-methoxy-1,4-benzodioxine (Compound 1)

### (Step A)

### (±)-5-[2-(3,5-Dichloro-4-pyridyl)-1-hydroxyethyl]-2,3-dihydro-8-methoxy-1,4-benzodioxine (Compound 1a)

A THF solution (10 ml) of diisopropylamine (2.3 ml) was cooled to -78°C in an argon atmosphere, and a 1.63 M hexane solution (10 ml) of butyl lithium was added dropwise thereto, followed by stirring at 0°C for 15 minutes. The mixture was again cooled to -78°C, and 3,5-dichloro-4-methylpyridine (2.6 g) was added thereto, followed by stirring at -78°C for 2 hours. The solution obtained was slowly added dropwise to a THF solution (20 ml) of Compound a (2.7 g) obtained in Reference Example 1, followed by stirring at -78°C for 2 hours and subsequently stirring at 0°C for 1 hour. A saturated aqueous ammonium chloride solution was added to the reaction solution, and the mixture was extracted with ether. The organic layer was washed with a saturated saline and dried over anhydrous magnesium sulfate, and the solvent was distilled off under reduced pressure to give Compound 1a as crude crystals (5.0 g, quantitatively).
NMR(CDCl₃, δ, ppm): 2.93(d, J=8Hz, 1H), 3.43(dd, J=6, 13Hz, 1H), 3.59(dd, J=9, 13Hz, 1H), 3.87(s, 3H), 4.23-4.35(m, 4H), 5.07-5.15(m, 1H), 6.46(d, J=9Hz, 1H), 6.76(d, J=9Hz, 1H), 8.41(s, 2H)
MASS (m/e): 355(M⁺)

### (Step B) Compound 1

Compound 1a (3.0 g) obtained in Step A was dissolved in acetone (30 ml), and Jones reagent (2.6 M) (4.9 ml) was added thereto at 0°C, followed by stirring at room temperature for 2 hours. The reaction solution was concentrated under reduced pressure, water was added to the residue, and the mixture was extracted with ethyl acetate. The organic layer was washed with a saturated saline and dried over anhydrous magnesium sulfate, and the solvent was distilled off under reduced pressure. The residue was purified by silica gel column chromatography (hexane/ethyl acetate = 4/1) to give Compound 1 (3.0 g, 75%) as colorless crystals.
Melting point: 158-160°C
NMR(CDCl₃, δ, ppm): 3.95(s, 3H), 4.42-4.49(m, 4H), 4.63(s, 2H), 6.61(d, J=9Hz, 1H), 7.55(d, J=9Hz, 1H), 8.49(s, 2H)
MASS (m/e): 353(M⁺)
IR (KBr, cm⁻¹): 1668, 1660, 1599, 1296, 1122

| Elemental analysis: C₁₆H₁₃Cl₂NO₄ | | | |
|---|---|---|---|
| Found (%): | C:54.21, | H:3.68, | N:3.77 |
| Calcd.(%): | C:54.26, | H:3.70, | N:3.95 |

### Example 2

### 2,3-Dihydro-8-methoxy-5-[1-oxo-2-(4-pyridyl)ethyl]-1,4-benzodioxine hydrochloride (Compound 2)

Compound 1 (1.1 g) obtained in Example 1 was dissolved in DMF (10 ml), and palladium-carbon (50%, containing water) (0.2 g) was added thereto, followed by stirring at room temperature for 24 hours under a hydrogen atmosphere. The reaction solution was filtered with celite, a saturated aqueous sodium bicarbonate solution was added to the filtrate, and the mixture was extracted with ethyl acetate. The organic layer was washed with a saturated saline and dried over anhydrous magnesium sulfate, and the solvent was distilled off under reduced pressure. The residue was purified by silica gel column chromatography (ethyl acetate) to give 2,3-dihydro-5-methoxy-8-[1-oxo-2-(4-pyridyl)ethyl]-1,4-benzodioxine (0.36 g, 41%) as colorless crystals. The compound obtained was dissolved in ethyl acetate, and a saturated ethyl acetate solution of hydrochloric acid was added thereto. The precipitated crystals were collected by filtration and washed with ethyl acetate to give Compound 2 as colorless crystals.
Melting point: 226-228°C
NMR(DMSO-d₆, δ, ppm): 3.84(s, 3H), 4.29-4.41(m, 4H), 4.66(s, H), 6.76(d, J=9Hz, 1H), 7.39(d, J=9Hz, 1H), 7.96(d, J=6Hz, 2H), 8.87(d, J=6Hz, 2H)
MASS (m/e): 285(M⁺)
IR (KBr, cm⁻¹): 1606, 1596, 1294, 1119

| Elemental analysis: C₁₆H₁₅NO₄·HCl·0.2H₂O | | | |
|---|---|---|---|
| Found (%): | C:59.06, | H:5.08, | N:4.30 |
| Calcd.(%): | C:59.07, | H:5.03, | N:4.28 |

### Example 3

### 5-[2-(2,6-Dichlorophenyl)-1-oxoethyl]-2,3-dihydro-8-methoxy-1,4-benzodioxine (Compound 3)

Compound c (1.0 g) obtained in Reference Example 3 was dissolved in methylene chloride (100 ml), and 2,6-dichlorophenylacetylchloride (6.73 g) was added thereto at room temperature. Titanium tetrachloride (5.3 ml) was added thereto under ice-cooling, followed by stirring for 15 hours. The reaction solution was added to an aqueous hydrochloric acid solution under ice-cooling, and the mixture was extracted with ethyl acetate. The organic layer was washed with a saturated saline and dried over anhydrous magnesium sulfate, and the solvent was distilled off under reduced pressure. The residue was purified by silica gel column chromatography (hexane/ethyl acetate = 3/1) to give Compound 3 (1.5 g, 72%) as colorless crystals.
Melting point: 154-155°C
NMR(CDCl₃, δ, ppm): 3.94(s, 3H), 4.07-4.23(m, 2H), 4.45-4.48(m, 2H), 4.64(s, 2H), 6.59(d, J=9Hz, 1H), 7.15(d, J=8Hz, 1H), 7.32(d, J=8Hz, 1H), 7.54(d, J=9Hz, 1H)
MASS (m/e): 352(M⁺)

| Elemental analysis: C₁₇H₁₄Cl₂O₄ | | | |
|---|---|---|---|
| Found (%): | C:57.59, | H:3.94, | N:0.05 |
| Calcd.(%): | C:57.81, | H:3.99, | N:0.00 |

### Example 4

### (E)-5-[2-(3,5-Dichloro-4-pyridyl)ethenyl]-2,3-dihydro-8-methoxy-1,4-benzodioxine (Compound 4)

p-Toluene sulfonic acid (0.8 g) was added to a toluene (20 ml) suspension of Compound 1a (1.6 g) obtained in Step A of Example 1, followed by refluxing under heating for 30 minutes. After being allowed to stand for cooling, a saturated aqueous sodium bicarbonate solution was added to the reaction solution for neutralization, and the mixture was extracted with ether. The organic layer was washed with a saturated saline and dried over anhydrous magnesium sulfate, and the solvent was distilled off under reduced pressure. The residue was purified by silica gel column chromatography (hexane/ethyl acetate = 3/1) to give Compound 4 (1.4 g, 95%) as yellow crystals.
Melting point: 94-95°C
NMR(CDCl₃, δ, ppm): 3.92(s, 3H), 4.37(s, 4H), 6.57(d, J=9Hz, 1H), 7.10(d, J=17Hz, 1H), 7.16(d, J=9Hz, 1H), 7.69(d, J=17Hz, 1H), 8.47(s, 2H)
MASS (m/e): 337(M⁺)
IR (KBr, cm⁻¹): 1603, 1500, 1290, 1120

| Elemental analysis: C₁₆H₁₃Cl₂NO₃ | | | |
|---|---|---|---|
| Found (%): | C:56.78, | H:3.85, | N:4.01 |
| Calcd.(%): | C:56.82, | H:3.87, | N:4.14 |

### Example 5

### 2,3-Dihydro-8-methoxy-5-[2-(4-pyridyl)ethyl]-1,4-benzodioxine hydrochloride (Compound 5)

Substantially the same procedure as in Example 2 was repeated using Compound 4 (0.8 g) obtained in Example 4 to give 2,3-dihydro-8-methoxy-5-[2-(4-pyridyl)ethyl]-1,4-benzodioxine (0.55 g, 86%) as colorless crystals. Furthermore, the compound was made into the hydrochloride according to a method similar to that in Example 2 to give Compound 5 as colorless crystals.
Melting point: 199-201°C
NMR(DMSO-d₆, δ, ppm): 2.86(t, J=7Hz, 3H), 3.11(t, J=7Hz, 3H), 3.69(s, 3H), 4.18(s, 4H), 4.66(s, 2H), 6.45(d, J=8Hz, 1H), 6.56(d, J=8Hz, 1H), 7.84(d, J=6Hz, 2H), 8.78(d, J=6Hz, 2H)
MASS (m/e): 271(M⁺)
IR (KBr, cm⁻¹): 1633, 1504, 1282, 1116

| Elemental analysis: C₁₆H₁₇NO₃·HCl | | | |
|---|---|---|---|
| Found (%): | C:62.12, | H:5.91, | N:4.47 |
| Calcd.(%): | C:62.44, | H:5.89, | N:4.55 |

### Example 6

### 5-(3,5-Dichloro-4-pyridylaminocarbonyl)-2,3-dihydro-8-methoxy-1,4-benzodioxine (Compound 6)

A mixture of Compound b (0.30 g) obtained in Reference Example 2, thionyl chloride (1.5 ml) and dichloromethane (3.0 ml) was refluxed under heating for 1 hour. After being allowed to stand for cooling, the solvent was distilled off, and the residue was dissolved in dried toluene. The solvent was distilled off under reduced pressure to remove the residual thionyl chloride to thereby give a crude acid chloride. 4-Amino-3,5-dichloropyridine (0.28 g) was dissolved in THF (8.0 ml), sodium hydride (140 mg) was added thereto under ice-cooling, followed by stirring at room temperature for 15 minutes, and then the mixture was again cooled with ice. A solution of the crude acid chloride obtained above dissolved in THF (5 ml) was added dropwise thereto under ice-cooling, followed by stirring for 1 hour under ice-cooling. The reaction mixture was extracted with ethyl acetate, the organic layer was washed with a saturated saline and dried over anhydrous magnesium sulfate, and the solvent was distilled off under reduced pressure. The residue was recrystallized from ethanol to give Compound 5 (0.22 g, 35%) as colorless crystals.
Melting point: 143-145°C
NMR(CDCl₃, δ, ppm): 3.96(s, 3H), 4.47-4.44(m, 2H), 4.56-4.53(m, 2H), 6.69(d, J=9Hz, 1H), 7.87(d, J=9Hz, 1H), 8.55(s, 2H), 9.49(brs, 1H)
MASS (m/e): 355(M⁺)
IR (KBr, cm⁻¹): 1689, 1492, 1297, 1116

| Elemental analysis: C₁₅H₁₂Cl₂N₂O₄ | | | |
|---|---|---|---|
| Found (%): | C:50.86, | H:3.46, | N:7.74 |
| Calcd.(%): | C:50.73, | H:3.41, | N:7.89 |

### Example 7

### 5-(2,6-Dichlorophenylaminocarbonyl)-2,3-dihydro-8-methoxy-1,4-benzodioxine (Compound 7)

Substantially the same procedure as in Example 6 was repeated using Compound b (0.60 g) obtained in Reference Example 2 and 2,6-dichloroaniline (0.55 g) to give Compound 7 (0.41 g, 41%) as colorless crystals.
Melting point: 176-177°C
NMR(CDCl₃, δ, ppm): 3.92 (s, 3H), 4.38-4.41(m, 2H), 4.48-4.51(m, 2H), 6.64(d, J=9Hz, 1H), 7.15(d, J=9Hz, 1H), 7.38(d, J=9Hz, 1H), 7.85(d, J=9Hz, 1H), 9.23(s, 1H)
MASS (m/e): 353(M⁺)

| Elemental analysis: C₁₆H₁₃Cl₂NO₄ | | | |
|---|---|---|---|
| Found (%): | C:54.14, | H:3.67, | N:3.83 |
| Calcd.(%): | C:54.25, | H:3.69, | N:3.94 |

### Example 8

### 5-(3-Ethoxycarbonylphenylaminocarbonyl)-2,3-dihydro-8-methoxy-1,4-benzodioine (Compound 8)

Substantially the same procedure as in Example 6 was repeated using Compound b (0.30 g) obtained in Reference Example 2 and ethyl 3-aminobenzoate (0.28 g) to give Compound 8 (0.39 g, 77%) as colorless crystals.
Melting point: 174-175°C
NMR(CDCl₃, δ, ppm): 1.41(t, J=7Hz, 3H), 3.92(s, 3H), 4.40(q, J=7Hz, 2H), 4.45-4.46(m, 2H), 4.54-4.57(m, 2H), 6.68(d, J=9Hz, 1H), 7.44 (t, J=8Hz, 1H), 7.80 (d, J=8Hz, 1H), 7.88(d, J=9Hz, 1H), 8.06(s, 1H), 8.15(d, J=8Hz, 1H), 9.54(s, 1H)
MASS (m/e): 357(M⁺)

| Elemental analysis: C₁₉H₁₉NO₆ | | | |
|---|---|---|---|
| Found (%): | C:63.64, | H:5,34, | N:3.83 |
| Calcd.(%): | C:63.85, | H:5.35, | N:3.91 |

### Example 9

### 5-(3-Carboxyphenylaminocarbonyl)-2,3-dihydro-8-methoxy-1,4-benzodioxine (Compound 9)

Compound 8 (0.19 g) obtained in Example 8 was suspended in ethanol (1 ml), and an aqueous 24% sodium hydroxide solution (1.3 ml) was added thereto, followed by refluxing under heating for 2 hours. After being allowed to stand for cooling, water was added thereto, and the pH was adjusted to 2 with an aqueous 2 N hydrochloric acid solution. The precipitated solid was collected by filtration to give Compound 9 (0.14 g, 80%) as white crystals.
Melting point: 283-284°C
NMR(CDCl₃, δ, ppm): 3.96(s, 3H), 4.44-4.46(m, 2H), 4.56-4.59(m, 2H), 6.71(d, J=9Hz, 1H), 7.46(t, J=8Hz, 1H), 7.79(d, J=9Hz, 1H), 7.83(d, J=8Hz, 1H), 8.06(d, J=8Hz, 1Hs 1H), 8.10(s, 1H).
MASS (m/e): 329(M⁺)

| Elemental analysis: C₁₇H₁₅NO₆ | | | |
|---|---|---|---|
| Found (%): | C:62.22, | H:4.58, | N:4.20 |
| Calcd.(%): | C:62.00, | H:4.59, | N:4.25 |

### Example 10

### 5-(4-Ethoxycarbonylphenyl)-2,3-dihydro-8-methoxy-1,4-benzodioxine (Compound 10)

### (Step A)

### 2,3-Dihydro-8-methoxy-5-tributylstanyl-1,4-benzodioxine (Compound 10a)

A THF solution (5 ml) of Compound aa (0.5 g) obtained in Reference Example 1 (Step A) was cooled to -78°C in an argon atmosphere, a 1.66 M hexane solution (1.48 ml) of butyl lithium was added dropwise thereto, followed by stirring at the same temperature for 30 minutes, and then tributyltin chloride (0.67 ml) was added thereto, followed by stirring for 5 minutes. The temperature was raised up to room temperature. An aqueous ammonium chloride solution and ether were added to the reaction solution, and the organic layer was separated. The organic layer was washed with a saturated saline and dried over anhydrous magnesium sulfate, and the solvent was distilled off under reduced pressure to give a crude objective compound. The obtained crude objective compound was immediately used in the next step without purification.

### (Step B) Compound 10

Compound 10a (0.93 g) obtained in Step A, ethyl 4-iodobenzoate (0.34 ml), palladium acetate (0.05 g), and sodium carbonate (0.54 g) were dissolved in DMF (10 ml), followed by stirring at 60°C for 6 hours. An aqueous ammonium fluoride solution and diethyl ether were added to the reaction solution, the solution was filtered with celite, and the organic layer was separated. The organic layer was washed with a saturated saline and dried over anhydrous magnesium sulfate, and the solvent was distilled off under reduced pressure. The residue was purified by silica gel column chromatography (hexane/ethyl acetate = 4/1) to give Compound 10 (0.30 g, 50%) as colorless crystals.
Melting point: 141-141.5°C
NMR(CDCl₃, δ, ppm): 0.92(t, J=7Hz, 3H), 3.93(s, 3H), 4.27-4.30(m, 2H), 4.35-4.38(m, 2H), 4.39(q, J=7Hz, 2H), 6.60(d, J=9Hz, 1H), 6.89(d, J=9Hz, 1H), 7.59(d, J=9Hz, 1H), 8.06(d, J=9Hz, 1H)
MASS (m/e): 314(M⁺)

| Elemental analysis: C₁₈H₁₈O₅ | | | |
|---|---|---|---|
| Found (%): | C:68.68, | H:5.93, | N:0.00 |
| Calcd.(%): | C:68.77, | H:5.77, | N:0.00 |

### Example 11

### 5-(4-Carboxyphenyl)-2,3-dihydro-8-methoxy-1,4-benzodioxine (Compound 11)

Substantially the same procedure as in Example 9 was repeated using Compound 10 (1.0 g) obtained in Example 10 to give Compound 11 (0.57 g, 63%) as white crystals.
Melting point: 281-284°C
NMR(DMSO-d₆, δ, ppm): 3.83(s, 3H), 4.22-4.38(m, 4H), 6.74(d, J=9Hz, 1H), 6.93(d, J=9Hz, 1H), 7.64(d, J=8Hz, 2H), 8.00(d, J=8Hz, 2H)
MASS (m/e): 286(M⁺)
IR (KBr, cm⁻¹): 1670, 1450, 1284, 1122

| Elemental analysis: C₁₆H₁₄O₅·0.3H₂O | | | |
|---|---|---|---|
| Found (%): | C:65.70, | H:4.97, | N:0.05 |
| Calcd.(%): | C:65.88, | H:5.04, | N:0.00 |

### Example 12

### 6-[2-(3,5-Dichloro-4-pyridyl)-1-oxoethyl]-3,4-dihydro-9-methoxy-2H-1,5-benzodioxepine (Compound 12)

### (Step A)

### (±)-6-[2-(3,5-Dichloro-4-pyridyl)-1-hydroxyethyl]-3,4-dihydro-9-methoxy-2H-1,5-benzodioxepine (Compound 12a)

Substantially the same procedure as in Step A of Example 1 was repeated using Compound d (3.2 g) obtained in Reference Example 4 to give Compound 12a (4.6 g, 81%) as colorless crystals.
NMR(CDCl₃, δ, ppm): 2.22-2.30(m, 2H), 2.93(d, J=8Hz, 1H), 3.26(dd, J=6, 13Hz, 1H), 3.56(dd, J=9Hz, 13Hz, 1H), 3.86(s, 3H), 4.25-4.35(m, H), 5.08-5.14(m, 1H), 6.46(d, J=9Hz, 1H), 6.76(d, J=9Hz, 1H), 8.41(s, 2H)
MASS (m/e): 369(M⁺)

### (Step B) Compound 12

Substantially the same procedure as in Step B of Example 1 was repeated using Compound 12a (3.0 g) obtained in Step A to give Compound 12 (2.2 g, 74%) as colorless crystals.
Melting point: 150-151°C
NMR(CDCl₃, δ, ppm): 2.30-2.36(m, 2H), 3.93(s, 3H), 4.36(t, J=6Hz, 2H), 4.46(t, J=6Hz, 2H), 4.65(s, 2H), 6.69(d, J=9Hz, 1H), 7.55(d, J=9Hz, 1H), 8.50(s, 2H)
MASS (m/e): 367(M⁺)
IR (KBr, cm⁻¹): 1670, 1583, 1286, 1105

| Elemental analysis: C₁₇H₁₅Cl₂NO₄ | | | |
|---|---|---|---|
| Found (%): | C:55.33, | H:4.06, | N:3.58 |
| Calcd.(%): | C:55.45, | H:4.11, | N:3.80 |

### Example 13

### 3,4-Dihydro-6-methoxy-9-[1-oxo-2-(4-pyridyl)ethyl]-2H-1,5-benzodioxepine hydrochloride (Compound 13)

Substantially the same procedure as in Example 2 was repeated using Compound 12 (1.2 g) obtained in Example 12 to give 3,4-dihydro-6-methoxy-9-[1-oxo-2-(4-pyridyl)ethyl]-2H-1,5-benzodioxepine (0.13 g, 13%) as colorless crystals. Furthermore, the compound was made into the hydrochloride according to a method similar to that in Example 2 to give Compound 13 as colorless crystals.
Melting point: 205-208°C
NMR(DMSO-d₆, δ, ppm): 2.12-2.20(m, 2H), 3.84(s, 3H), 4.12(t, J=6Hz, 2H), 4.31(t, J=6Hz, 2H), 4.67(s, 2H), 6.89(d, J=9Hz, 1H), 7.45(d, J=9Hz, 1H), 7.93(d, J=6Hz, 2H), 8.85(d, J=6Hz, 2H)
MASS (m/e): 299(M⁺)
IR (KBr, cm⁻¹): 1657, 1587, 1298, 1108

| Elemental analysis: C₁₇H₁₇NO₄·HCl·H₂O | | | |
|---|---|---|---|
| Found (%): | C:57.69, | H:5.83, | N:3.75 |
| Calcd.(%): | C:57.71, | H:5.70, | N:3.96 |

### Example 14

### (E)-6-[2-(3,5-Dichloro-4-pyridyl)ethenyl]-3,4-dihydro-9-methoxy-2H-1,5-benzodioxepine (Compound 14)

Substantially the same procedure as in Example 4 was repeated using Compound 12a (1.4 g) obtained in Step A of Example 12 to give Compound 14 (1.1 g, 79%) as yellow crystals.
Melting point: 118-120°C
NMR(CDCl₃, δ, ppm): 2.22-2.29(m, 2H), 3.90(s, 3H), 4.31-4.35(m, 4H), 6.65(d, J=9Hz, 1H), 7.05(d=17Hz, 1H), 7.26(d, J=9Hz, 1H), 7.76(d, J=17Hz, 1H), 8.47(s, H)
MASS (m/e): 351(M⁺)
IR (KBr, cm⁻¹): 1593, 1502, 1297, 1101

| Elemental analysis: C₁₇H₁₅Cl₂NO₃ | | | |
|---|---|---|---|
| Found (%): | C:57.99, | H:4.28, | N:3.87 |
| Calcd.(%): | C:57.97, | H:4.29, | N:3.98 |

### Example 15

### 3,4-Dihydro-6-methoxy-9-[2-(4-pyridyl)ethyl]-2H-1,5-benzodioxepine hydrochloride (Compound 15)

Substantially the same procedure as in Example 2 was repeated using Compound 14 (0.57 g) obtained in Example 14 to give 3,4-dihydro-6-methoxy-9-[2-(4-pyridyl)ethyl]-2H-1,5-benzodioxepine (0.33 g, 71%) as colorless crystals. Furthermore, the compound was made into the hydrochloride according to a method similar to that in Example 2 to give Compound 15 as colorless crystals.
Melting point: 185-187°C
NMR(DMSO-d₆, δ, ppm): 2.06-2.11(m, 2H), 2.88(t, J=7Hz, 3H), 3.08(t, J=7Hz, 3H), 3.70(s, 3H), 4.02-4.08(m, 4H), 6.59(d, J=8Hz, H), 6.73(d, J=8Hz, H), 7.84(d, J=6Hz, H), 8.77 (d, J=6Hz, 2H)
MASS (m/e): 285(M⁺)
IR (KBr, cm⁻¹): 1633, 1497, 1257, 1101

| Elemental analysis: C₁₇H₁₉NO₃·HCl | | | |
|---|---|---|---|
| Found (%): | C:63.12, | H:6.29, | N:4.25 |
| Calcd.(%): | C:63.45, | H:6.26, | N:4.35 |

### Example 16

### 6-(3,5-Dichloro-4-pyridylaminocarbonyl)-3,4-dihydro-9-methoxy-2H-1,5-benzodioxepine (Compound 16)

Substantially the same procedure as in Example 6 was repeated using Compound e (0.70 g) obtained in Reference Example 5 to give Compound 16 (0.66 g, 56%) as colorless crystals.
Melting point: 149-150°C
NMR(CDCl₃, δ, ppm): 2.38-2.34(m, 2H), 3.94(s, 3H), 4.36(t, J=6Hz, 2H), 4.54(t, J=6Hz, 2H), 6.77(d, J=9Hz, 1H), 7.95(d, J=9Hz, 1H), 8.55(s, 2H), 9.92(brs, 1H)
MASS (m/e): 369(M⁺)
IR (KBr, cm⁻¹): 1689, 1486, 1270, 1091

| Elemental analysis: C₁₆H₁₄Cl₂N₂O₄·0.4H₂O | | | |
|---|---|---|---|
| Found (%): | C:51.04, | H:3.79, | N:7.39 |
| Calcd.(%): | C:51.06, | H:3.96, | N:7.44 |

### Example 17

### 7-[2-(3,5-Dichloro-4-pyridyl)-1-oxoethyl]-10-methoxy-2,3,4,5-tetrahydro-1,6-benzodioxocine (Compound 17)

Compound f (1.0 g) obtained in Reference Example 6 was dissolved in DMF (10 ml), and 1,4-dibromobutane (0.45 ml) and potassium carbonate (0.95 g) were added thereto, followed by stirring at 60°C for 1 hour. After being allowed to stand for cooling, water was added, and the mixture was extracted with ether. The organic layer was washed with a saturated saline and dried over sodium sulfate, and the solvent was distilled off under reduced pressure. The residue was purified by silica gel column chromatography (hexane/ethyl acetate = 3/1), and recrystallized from ethanol to give Compound 17 (0.36 g, 30%) as white crystals.
Melting point: 142-143°C
NMR(CDCl₃, δ, ppm): 1.87-2.13(m, 4H), 3.92(s, 3H), 4.27-4.40(m, 2H), 4.55-4.72(m, 4H), 6.67(d, J=9Hz, 1H), 7.58(d, J=9Hz, 1H), 8.49(s, 2H)
MASS (m/e): 381(M⁺)

| Elemental analysis: C₁₈H₁₇Cl₂NO₄ | | | |
|---|---|---|---|
| Found (%): | C:56.63, | H:4.49, | N:3.57 |
| Calcd.(%): | C:56.56, | H:4.48, | N:3.66 |

### Example 18

### 7-[2-(3,5-Dichloro-N-oxo-4-pyridyl)-1-oxoethyl]-10-methoxy-2,3,4,5-tetrahydro-1,6-benzodioxocine (Compound 18)

Compound 17 (1.4 g) obtained in Example 17 was dissolved in dichloromethane (14 ml), and m-chloroperbenzoic acid (1.2 g) was added thereto, followed by stirring at room temperature for 1 hour. Further, m-chloroperbenzoic acid (1.2 g) was added thereto, followed by stirring for 5 hours. An aqueous sodium bisulfite solution and a saturated aqueous sodium bicarbonate solution were added thereto, and the mixture was extracted with ethyl acetate. The organic layer was washed with a saturated saline and dried over sodium sulfate, and the solvent was distilled off under reduced pressure. The residue was purified by silica gel column chromatography (chloroform/methanol = 30/1), and recrystallized from ethanol to give Compound 18 (0.85 g, 59%) as white crystals.
Melting point: 188-189°C
NMR(DMSO-d₆, δ, ppm): 1.65-2.00(m, 4H), 3.85(s, 3H), 4.05-4.23(m, 2H), 4.44-4.55(m, 4H), 6.86(d, J=9Hz, 1H), 7.48(d, J=9Hz, 1H), 8.64(s, 2H)
MASS (m/e): 397(M⁺)
IR (KBr, cm⁻¹): 1664, 1581, 1433, 1290, 1105

| Elemental analysis: C₁₈H₁₇Cl₂NO₅ | | | |
|---|---|---|---|
| Found (%): | C:54.24, | H:4.43, | N:3.35 |
| Calcd.(%): | C:54.29, | H:4.30, | N:3.52 |

### Example 19

### 7-(3,5-Dichloro-4-pyridylaminocarbonyl)-10-methoxy-2,3,4,5-tetrahydro-1,6-benzodioxocine (Compound 19)

Substantially the same procedure as in Example 5 was repeated using Compound g (1.1 g) obtained in Reference Example 7 to give Compound 19 (1.0 g, 56%) as white crystals.
Melting point: 115-117°C
NMR(DMSO-d₆, δ, ppm): 1.64-1.96(m, 4H), 3.84(s, 3H), 4.08-4.23(m, 2H), 4.45-4.59(m, 2H), 6.89(d, J=9Hz, 1H), 7.59(d, J=9Hz, 1H), 8.72(s, 2H), 10.1(s, 1H)
MASS (m/e): 382(M⁺)

| Elemental analysis: C₁₇H₁₆Cl₂N₂O₄ | | | |
|---|---|---|---|
| Found (%): | C:53.33, | H:4.28, | N:7.22 |
| Calcd.(%): | C:53.28, | H:4.21, | N:7.31 |

### Reference Example 1

### 2,3-Dihydro-8-methoxy-1,4-benzodioxine-5-carbaldehyde (Compound a)

### (Step A)

### 5-Bromo-2,3-dihydro-8-methoxy-1,4-benzoxine (Compound aa)

3-Bromo-6-methoxycatechol (16 g) was dissolved in DMF (50 ml), and 1,2-dibromoethane (15 ml) and potassium fluoride (21 g) were added thereto, followed by stirring under heating at 110°C for 6 hours. After being allowed to stand for cooling, water was added thereto, and the mixture was extracted with ether. The organic layer was washed with an aqueous sodium hydroxide solution, water and a saturated saline in this order and dried over anhydrous magnesium sulfate, and the solvent was distilled off under reduced pressure. The residue was purified by silica gel column chromatography (hexane/ethyl acetate = 4/1) to give Compound aa (5.9 g, 34%) as a white solid.
NMR(CDCl₃, δ, ppm): 3.87(s, 3H), 4.32-4.38(m, 4H), 6.42(d, J=9Hz, 1H), 7.04(d, J=9Hz, 1H)
MASS (m/e): 244(M⁺)

### (Step B) Compound a

A THF solution (60 ml) of Compound aa (6.1 g) obtained in Step A of Reference Example 1 was cooled to -78°C in an argon atmosphere, and a 1.63 M hexane solution (17 ml) of butyl lithium was added dropwise thereto, followed by stirring at the same temperature for 30 minutes. Dimethylformamide (3.9 ml) was slowly added dropwise to the reaction solution, followed by stirring at -78°C for 15 minutes and subsequently stirring at room temperature for 1 hour. A saturated aqueous ammonium chloride solution was added to the reaction solution, and the mixture was extracted with ether. The organic layer was washed with a saturated saline and dried over anhydrous magnesium sulfate, and the solvent was distilled off under reduced pressure. The residue was purified by silica gel column chromatography (hexane/ethyl acetate = 3/1) to give Compound a (2.8 g, 57%) as colorless crystals.
NMR(CDCl₃, δ, ppm): 3.95(s, 3H), 4.39(s, 4H), 6.60(d, J=9Hz, 1H), 7.44(d, J=9Hz, 1H), 10.21(s, 1H)
MASS (m/e): 194(M⁺)

### Reference Example 2

### 2,3-Dihydro-8-methoxy-1,4-benzodioxine-5-carboxylic acid (Compound b)

A THF solution (30 ml) of Compound aa (1.3 g) obtained in Step A of Reference Example 1 was cooled to -78°C, and a 1.69 M hexane solution (8.2 ml) of butyl lithium was added dropwise thereto, followed by stirring at the same temperature for 30 minutes. Dry ice was added to the reaction solution, followed by stirring at room temperature for 1 hour, water is added to the reaction solution, and the mixture was washed with ether. A 6 N aqueous hydrochloric acid solution was added to the water layer, and a precipitated solid was collected by filtration to give Compound b (0.63 g, 57%) as a colorless solid.
NMR(CDCl₃, δ, ppm): 3.95(s, 3H), 4.41-4.44(m, 2H), 4.50-4.53(m, 2H), 6.65(d, J=9Hz, 1H), 7.75(d, J=9Hz, 1H)
MASS (m/e): 210(M⁺)

### Reference Example 3

### 2,3-Dihydro-8-methoxy-1,4-benzodioxine (Compound c)

Substantially the same procedure as in Step A of Reference Example 1 was repeated using 3-methoxycatechol (3.0 g) to give Compound c (2.28 g, 64.2%) as white oily substances.
NMR(CDCl₃, δ, ppm): 3.86(s, H), 4.23-4.26(m, 2H), 4.29-4.32(m, 2H), 6.50(d, J=8Hz, 1H), 6.52(d, J=8Hz, 1H), 6.76(t, J=8Hz, 1H)
MASS (m/e): 166(M⁺)

### Reference Example 4

### 3,4-Dihydro-9-methoxy-2H-1,5-benzodioxepine-6-carbaldehyde (Compound d)

### (Step A)

### 6-Bromo-3,4-dihydro-9-methoxy-2H-1,5-benzodioxepine (Compound da)

3-Bromo-6-methoxycatechol (31 g) was dissolved in DMF (50 ml), and 1,3-dibromopropane (36 ml) and potassium fluoride (42 g) were added thereto, followed by stirring under heating at 110°C for 12 hours. After being allowed to stand for cooling, water was added thereto, and the mixture was extracted with ether. The organic layer was washed with an aqueous sodium hydroxide solution, water and a saturated saline in this order and dried over magnesium sulfate, and the solvent was distilled off under reduced pressure. The residue was purified by silica gel column chromatography (hexane/ethyl acetate = 7/1) to give Compound da (6.7 g, 18%) as a white solid.
NMR(CDCl₃, δ, ppm): 2.37-2.41(m, 2H), 3.98(s, 3H), 4.34-4.45(m, 4H), 6.64(d, J=9Hz, 1H), 7.26(d, J=9Hz, 1H)
MASS (m/e): 258(M⁺)

### (Step B) Compound d

Substantially the same procedure as in Step B of Reference Example 1 was repeated using Compound da (6.5 g) obtained in Step A of Reference Example 4 to give Compound d (3.3 g, 63%) as colorless crystals.
NMR(CDCl₃, δ, ppm): 2.26-2.35(m, 2H), 3.93(s, 3H), 4.35(t, J=6Hz, 2H), 4.41(t, J=6Hz, 2H), 6.67(d, J=9Hz, 1H), 7.53(d, J=9Hz, 1H), 10.28(s, 1H)
MASS (m/e): 208(M⁺)

### Reference Example 5

### 3,4-Dihydro-9-methoxy-2H-1,5-benzodioxepine-6-carboxylic acid (Compound e)

Substantially the same procedure as in Reference Example 2 was repeated using Compound da (3.0 g) obtained in Step A of Reference Example 4 to give Compound e (0.80 g, 31%) as white crystals.
NMR(CDCl₃, δ, ppm): 2.32-2.40(m, 2H), 3.93(s, 3H), 4.35(t, J=6Hz, 2H), 4.53(t, J=6Hz, 2H), 6.75(d, J=9Hz, 1H), 7.85(d, J=9Hz, 1H)
MASS (m/e): 224(M⁺)

### Reference Example 6

### [2-(3,5-Dichloro-4-pyridyl)-1-oxoethyl]-2,3-dihydroxy-4-methoxybenzene (Compound f)

### (Step A)

### Methyl 2,3,4-trimethoxybenzoate (Compound fa)

2,3,4-Trimethoxybenzoic acid (7.3 g) was dissolved in methanol (73 ml), and sulfuric acid (15 ml) was added thereto, followed by stirring under heating at 80°C for 3 hours. After being allowed to stand for cooling, the solvent was distilled off under reduced pressure, the residue was added to cold water, and the mixture was extracted with ethyl acetate. The organic layer was washed with a saturated aqueous sodium bicarbonate solution and a saturated saline in this order and dried over anhydrous sodium sulfate, and the solvent was distilled off under reduced pressure to give Compound fa (6.8 g, 87%) as colorless oily substances.
NMR(CDCl₃, δ, ppm): 3.88(s, 3H), 3.89(s, 3H), 3.91(s, 3H), 3.94(s, 3H), 6.70(d, J=9Hz, 1H), 7.61(d, J=9Hz, 1H)
MASS (m/e): 226(M⁺)

### (Step B)

### [2-(3,5-Dichloro-4-pyridyl)-1-oxoethyl]-2,3,4-trimethoxybenzene (Compound fb)

A THF solution (30 ml) of diisopropylamine (9.9 ml) was cooled to -78°C in an argon atmosphere, and a 1.66 M hexane solution (46 ml) of butyl lithium was added dropwise thereto, followed by stirring at 0°C for 15 minutes. The mixture was again cooled to -78°C, and 3,5-dichloro-4-methylpyridine (12 g) was added thereto, followed by stirring at -78°C for 2 hours. The obtained solution was slowly added dropwise to a THF solution (40 ml) of Compound fa (5.7 g) obtained in Step A, followed by stirring at -78°C for 2 hours and subsequently stirring at 0°C for 1 hour. A saturated aqueous ammonium chloride solution was added to the reaction solution, and the mixture was extracted with ether. The organic layer was washed with a saturated saline and dried over anhydrous sodium sulfate, and the solvent was distilled off under reduced pressure. The residue was recrystallized from ethyl acetate/diisopropyl ether to give Compound fb (7.0 g, 78%) as pale yellow crystals.
Melting point: 125-128°C
NMR(CDCl₃, δ, ppm): 3.92 (s, 3H), 3.94(s, 3H), 4.13(s, 3H), 4.69(s, 2H), 6.77(d, J=9Hz, 1H), 7.63(d, J=9Hz, 1H), 8.50(s, 2H)
MASS (m/e): 355(M⁺)

### (Step C)

### [2-(3,5-Dichloro-4-pyridyl)-1-oxoethyl]-2,3-dihydroxy-4-methoxybenzene (Compound f)

Compound fb (4.0 g) obtained in Step B was dissolved in dichloromethane (60 ml), and a 1.0 M dichloromethane solution (23 ml) of boron trichloride was added thereto, followed by stirring for 10 minutes. A 1.0 M dichloromethane solution (23 ml) of boron trichloride was again added thereto, followed by stirring for 24 hours. The reaction solution was added to a 5% aqueous sodium hydroxide solution at 0°C, the pH was adjusted to 2 with sulfuric acid, and the mixture was extracted with ethyl acetate. The organic layer was washed with a saturated saline and dried over anhydrous sodium sulfate, and the solvent was distilled off under reduced pressure to give Compound f (3.2 g, 86%) as a pale yellow solid.
Melting point: 180-183°C
NMR(CDCl₃, δ, ppm): 4.01(s, 3H), 4.68(s, 2H), 6.61(d, J=9Hz, 1H), 7.51(d, J=9Hz, 1H) ,8.55(s, 2H), 11.9(s, 1H)

### Reference Example 7

### 10-Methoxy-2,3,4,5-tetrahydro-1,6-benzodioxocine-7-carboxylic acid (Compound g)

### (Step A)

### 7-Bromo-10-methoxy-2,3,4,5-tetrahydro-1,6-benzodioxocine (Compound ga)

Substantially the same procedure as in Example 17 was repeated using 3-bromo-6-methoxycatechol (5.4 g) to give Compound ga (2.9 g, 44%) as colorless oily substances.
NMR(DMSO-d₆, δ, ppm): 1.65-1.85(m, 4H), 3.76(s, 3H), 4.07-4.17(m, 2H), 4.18-4.28(m, 2H), 6.72(d, J=9Hz, 1H), 7.24(d, J=9Hz, 1H)
MASS (m/e): 273(M⁺)

### (Step B)

### 10-Methoxy-2,3,4,5-tetrahydro-1,6-benzodioxocine-7-carboxylic acid

Substantially the same procedure as in Reference Example 2 was repeated using Compound ga (3.3 g) obtained in Step A to give Compound g (1.3 g, 45%) as a white solid.
NMR(DMSO-d₆, δ, ppm): 1.63-1.84(m, 4H), 3.82(s, 3H), 4.05-4.15(m, 2H),4.15-4.31(m, 2H), 6.83(d, J=9Hz, 1H), 7.50(d, J=9Hz, 1H), 12.4(s, 1H)
MASS (m/e): 238(M⁺)

### Preparation Example 1 Tablet

Tablets having the following composition are prepared according to a conventional method.

| | |
|---|---|
| Compound 1 | 50 mg |
| Lactose | 60 mg |
| Potato starch | 50 mg |
| Polyvinyl alcohol | 2 mg |
| Magnesium stearate | 1 mg |
| Tar dye | a trace amount |

### Preparation Example 2 Powder

Powder having the following composition is prepared according to a conventional method.

| | |
|---|---|
| Compound 1 | 50 mg |
| Lactose | 250 mg |

### Preparation Example 3 Nasal inhalation

A nasal inhalation having the following composition is prepared according to a conventional method.

| | |
|---|---|
| Compound 1 | 1 mg |
| Lactose | 20 mg |

### Preparation Example 4 Ophthalmic preparation

An ophthalmic preparation having the following composition is prepared according to a conventional method.

| | |
|---|---|
| Compound 1 | 10 mg |
| Sodium chloride | 20 mg |
| Methylparaben | 0.1 mg |
| Propylparaben | 0.1 mg |
| Injectable water | q.s. (total 1.0 ml) |

### Preparation Example 5 Transdermal therapeutic system

A transdermal therapeutic system having the following composition is prepared according to a conventional method.

| | |
|---|---|
| Compound 1 | 10 g |
| White beeswax | 80 g |
| Stearyl alcohol | 30 g |
| Cholesterol | 30 g |
| White vaseline | q.s. (total 1,000 g) |

### Preparation Example 6 Suppository

A suppository having the following composition is prepared according to a conventional method.

| | |
|---|---|
| Compound 1 | 10 mg |
| Witepsol W-15 | 1.79 g |

### Preparation Example 7 Injectable preparation

An Injectable preparation having the following composition is prepared according to a conventional method.

| | |
|---|---|
| Compound 1 | 10 mg |
| Injectable water | q.s. (total 1.0 ml) |

### Preparation Example 8 Syrup

A syrup having the following composition is prepared according to a conventional method.

| | |
|---|---|
| Compound 1 | 10 mg |
| Sucrose | 300 mg |
| Methylparaben | 0.5 mg |
| Sodium benzoate | 0.5 mg |
| Lemon flavor | q.s. |
| Dye | q.s. |
| Purified water | q.s. (total 1.0 ml) |

### Preparation Example 9 Nasal spray

A nasal spray having the following composition is prepared according to a conventional method.

| | |
|---|---|
| Compound 1 | 10 mg |
| Sodium chloride | 8 mg |
| Benzalkonium chloride | 0.1 mg |
| Carbopol | 10 mg |
| Purified water | q.s. (total 1.0 ml) |

### Preparation Example 10 Tablet

Tablets having the following composition are prepared according to a conventional method.

| | |
|---|---|
| Compound 1 | 10 mg |
| Lactose | 140 mg |
| Corn starch | 45 mg |
| Sodium croscarmellose | 10 mg |
| Hydroxypropyl cellulose L | 4 mg |
| Magnesium stearate | 1 mg |

### Preparation Example 11 Capsule

Capsules having the following composition are prepared according to a conventional method.

| | |
|---|---|
| Compound 1 | 10 mg |
| Lactose | 185 mg |
| Sodium croscarmellose | 10 mg |
| Hydroxypropyl cellulose L | 4 mg |
| Magnesium stearate | 1 mg |

### Preparation Example 12 Dry syrup

A dry syrup having the following composition is prepared according to a conventional method.

| | |
|---|---|
| Compound 1 | 10 mg |
| Sucrose | 0.7 g |
| D-Mannitol | 0.28 g |
| Pullulan | 20 mg |

### Preparation Example 13 Granules

Granules having the following composition are prepared according to a conventional method.

| | |
|---|---|
| Compound 1 | 10 mg |
| Lactose | 0.8 g |
| Corn starch | 0.17 g |
| Hydroxypropyl cellulose L | 30 mg |

### Industrial Applicability

The present invention can provide oxygen-containing heterocyclic compounds which exhibit PDE IV inhibitory activity and which are useful as therapeutic agents for asthma, allergy, rheumatoid arthritis, psoriasis, myocardial infarction, depression, amnesia, multiple sclerosis, Crohn's disease, systemic lupus erythematosus, diabetes, wounds, AIDS, and the like.

## Claims

1. An oxygen-containing heterocyclic compound represented by following Formula (I): 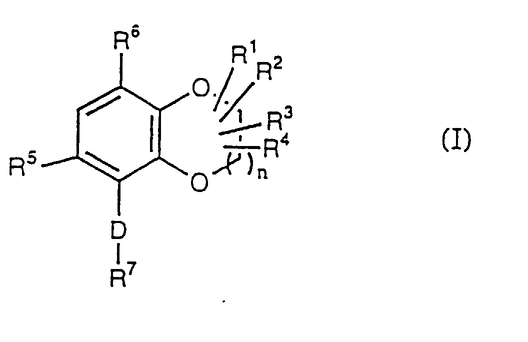 wherein n represents an integer of 1 to 4; R¹, R², R³ and R⁴ are the same or different and represent hydrogen, substituted or unsubstituted lower alkyl, substituted or unsubstituted cycloalkyl, polycycloalkyl, substituted or unsubstituted lower alkenyl, substituted or unsubstituted cycloalkenyl, substituted or unsubstituted aryl, a substituted or unsubstituted aromatic heterocyclic group, or substituted or unsubstituted aralkyl, or two groups present on the same carbon atom among R¹, R², R³ and R⁴ are combined to represent a saturated carbon ring, two groups present on the adjacent carbon atoms among R¹, R², R³ and R⁴ are combined to represent a saturated carbon ring together with the two carbon atoms adjacent thereto, or two groups present on the adjacent carbon atoms among R¹, R², R³ and R⁴ are combined to represent a single bond; R⁵ represents hydrogen or halogen; R⁶ represents hydroxy or substituted or unsubstituted lower alkoxy; D represents (1) -C(R⁸)(R⁹)-X- [wherein R⁸ and R⁹ are the same or different and represent hydrogen, substituted or unsubstituted lower alkyl, cycloalkyl, polycycloalkyl, substituted or unsubstituted lower alkenyl, cycloalkenyl, substituted or unsubstituted aryl, a substituted or unsubstituted aromatic heterocyclic group, hydroxy, substituted or unsubstituted lower alkoxy, lower alkanoyloxy, substituted or unsubstituted lower alkanoyl, cycloalkylcarbonyl, lower alkoxycarbonyl, cyano or halogen, or R⁸ and R⁹ are combined to represent O, S or NR¹⁰ (wherein R¹⁰ represents hydrogen, substituted or unsubstituted lower alkyl, cycloalkyl, polycycloalkyl, substituted or unsubstituted aryl, a substituted or unsubstituted aromatic heterocyclic group, hydroxy, substituted or unsubstituted lower alkoxy, or lower alkanoyloxy); X represents -C(R¹¹)(R¹²)- (wherein R¹¹ and R¹² are the same or different and represent hydrogen, substituted or unsubstituted lower alkyl, cycloalkyl, polycycloalkyl, substituted or unsubstituted lower alkenyl, cycloalkenyl, substituted or unsubstituted aryl, a substituted or unsubstituted aromatic heterocyclic group, substituted or unsubstituted lower alkanoyl, cycloalkylcarbonyl, lower alkoxycarbonyl, or cyano, or represent a single bond together with R⁸), S, NR¹³ (wherein R¹³ represents hydrogen, substituted or unsubstituted lower alkyl, cycloalkyl, substituted or unsubstituted aryl, a substituted or unsubstituted aromatic heterocyclic group, or substituted or unsubstituted aralkyl, or represents a single bond together with R⁸), or a bond], or (2) a bond; R⁷ represents (1) substituted or unsubstituted lower alkyl, substituted or unsubstituted cycloalkyl, polycycloalkyl, substituted or unsubstituted aryl, substituted or unsubstituted aralkyl, a substituted or unsubstituted aromatic heterocyclic group, a substituted or unsubstituted heterocyclic group, or pyridine-*N*-oxide, (2) -Y-ZR¹⁴ [wherein Y represents substituted or unsubstituted aryl, or a substituted or unsubstituted aromatic heterocyclic group; Z represents O, S, or NR¹⁵ (wherein R¹⁵ represents hydrogen, or substituted or unsubstituted lower alkyl, or represents a substituted or unsubstituted heterocyclic group together with R¹⁴); and R¹⁴ represents substituted or unsubstituted lower alkyl, substituted or unsubstituted aryl, or a substituted or unsubstituted aromatic heterocyclic group, or represents a substituted or unsubstituted heterocyclic group together with R¹⁵)], (3) -Y-Z-(CH₂)ₘ-N(R^{16a})R^{16b} (wherein Y and Z have the same meanings as defined above; R^{16a} and R^{16b} are the same or different and represent hydrogen, or substituted or unsubstituted lower alkyl, or R^{16a} and R^{16b} are combined to represent a substituted or unsubstituted heterocyclic group; and m represents an integer of 1 to 4), or (4) -Y-CON(R^{17a})R^{17b} (wherein Y has the same meaning as defined above; and R^{17a} and R^{17b} are the same or different and represent hydrogen, or substituted or unsubstituted lower alkyl, or R^{17a} and R^{17b} are combined to represent a substituted or unsubstituted heterocyclic group), or a pharmaceutically acceptable salt thereof.
